# EUROPEAN PATENT APPLICATION

(11) **EP 4 628 105 A2**
(43) Date of publication of application: **08.10.2025**
(21) Application number: 25168122.7
(22) Date of filing: 03.02.2017
(51) Int. Cl.: A61K 47/68

(54) **MULTISPECIFIC ANTIGEN-BINDING MOLECULE WITH IMPROVED INTERNALIZATION CHARACTERISTICS**

(30) Priority: 05.02.2016 DK PA201600074
(62) Divisional of application: 17704190.2
(71) Applicant: Genmab A/S, 2500 Valby (DK)
(72) Inventor: DE GOEIJ, Bart, 3584 CT Utrecht (NL); MELIS, Joost, 3584 CT Utrecht (NL); VINK, Tom, 6285 AZ Epen (NL); TEN NAPEL, Hendrik, 3584 CT Utrecht (NL); BREIJ, Esther, 3584 CT Utrecht (NL); SATIJN, David, 3584 CT Utrecht (NL); PARREN, Paul, 3984 PR Odijk (NL)
(74) Representative: Inspicos P/S

(57) **Abstract**

The present invention relates to a multispecific antigen-binding molecule comprising a first antigen-binding domain and a second antigen-binding domain. The first domain specifically binds a target molecule (T), and the second domain specifically binds an internalizing effector protein (E), the second antigen-binding domain having a dissociation constant (K_{D}) with E of between 10⁻⁹ and 10⁻⁸ M. The multispecific antigen-binding molecule is useful in a method for treating and/or preventing a cancer.

## Description

### FIELD OF THE INVENTION

The present invention relates to a multispecific antigen-binding molecule, compositions comprising said multispecific antigen-binding molecule, and the use of said multispecific antigen-binding molecule in the treatment of a disease.

### BACKGROUND OF THE INVENTION

Since the development of the first monoclonal antibodies research has been directed at further optimization of antibodies for human therapy. The first monoclonal antibodies originated from mice and rats. The progress in antibody technology has led to the availability of humanized and human antibodies with decreased immunogenicity risk profiles. Genetic and chemical engineering is leading to the development of more potent antibodies with an increased therapeutic potential. This includes antibodies with optimized Fc-mediated effector functions, optimized binding characteristics or optimized anti-tumor activity through the conjugation to toxic molecules (antibody-drug conjugates).

Antibody-drug conjugates (ADC) are emerging as powerful therapeutics for the treatment of cancer, as they combine antibody-mediated tumor-targeting with the cytotoxic activity of toxins. ADCs comprise an antibody (e.g. a monoclonal antibody, a single-chain variable fragment [scFv], or a bispecific antibody) linked to a cytotoxic payload or drug. The advantage of directing cytotoxic drugs to tumors with an antibody against a tumor-associated antigen is that the therapeutic window can be improved relative to the unconjugated cytotoxic drug. This allows for the application of cytotoxic payloads of increased potency.

Currently, two ADCs have already been approved for therapeutic use: brentuximab vedotin (Adcetris) for the treatment of relapsed Hodgkin lymphoma and relapsed sALCL, and trastuzumab emtansine (Kadcyla), for the treatment of HER2-positive, metastatic breast cancer patients who previously received trastuzumab and a taxane, separately or in combination. In addition, over 50 different ADCs are currently in clinical evaluation. In many cases, ADCs rely on internalization of the toxin-conjugated antibody molecules into targeted cells to release their payload and induce subsequent cytotoxicity. Most ADCs in clinical development are designed to be stable in circulation and to release their cytotoxic payload after internalization and lysosomal processing of the antigen/ADC complex.

However, the requirement for antigen- and antibody-mediated internalization limits the number of suitable ADC targets. Many tumor-associated antigens do not internalize well or do not route well to lysosomes and therefore represent less promising candidate targets for ADC-based therapeutics. Also, in many cases, intracellular processing of ADCs is inefficient. Following internalization, receptors such as transferrin, HER2, cell adhesion molecule L1 and integrins, are continuously recycled back from the endosomal compartment to the plasma membrane. High antigen turn over, efficient transloction to the lysosomes and highly toxic payloads are therefore required to achieve maximal killing activity by ADCs.

Methods to enhance internalization, lysosomal targeting, intratumoral and intracellular processing of ADCs might be used to enhance the tumor cell killing activity of ADCs. One approach to optimize the ADCs activity is by selecting a specific epitope on the tumor-associated target, as the specific epitope recognized may influence internalization and lysosomal routing. For instance, it has been previously shown that the efficacy of HER2-ADCs can be improved by selecting HER2-ADCs that allow enhanced internalization by piggybacking of HER2 onto other ErbB molecules via heterodimer formation. This provides an attractive strategy for increasing ADC delivery and tumor cell killing capacity to both high and low HER2 expressing tumor cells.

Generally, efficient internalization of the ADC, followed by routing to lysosomes, where proteolysis can take place, is preferred. For many cell surface proteins and carbohydrate structures on tumor cells, however, the magnitude of these processes is insufficient to allow sufficiently potent cell killing by the ADC.

International patent application WO2013/138400 describes multispecific antibodies having a first domain that specifically binds a target antigen such as IL-4R or SOST, and a second domain that specifically binds an internalizing effector protein. If the target antigen is a tumor-associated antigen, the binding of the tumor-associated antigen and the internalizing effector protein by the multispecific antibody facilitates the targeted killing of tumor cells.

It is an object of the present invention to provide bispecific or multispecific antigen-binding molecules for which the internalizing capacity is increased relative to the monospecific antigen-binding molecule against a tumor-associated target.

It is another object of the present invention to provide bispecific or multispecific antibody drug conjugate molecules for which the internalizing capacity is increased relative to the monospecific antibody drug conjugate molecule directed against a tumor-associated target.

It is another object of the present invention to provide ADCs with increased internalization, lysosomal targeting and/or intracellular processing in tumor cells.

It is another object of the present invention to provide ADCs with increased cytotoxicity to tumor cells and/or fewer side effects.

It is another object of the present invention to provide ADCs with an increased therapeutic window.

It is another object of the present invention to enable the generation of effective ADCs against tumor-associated antigens which internalize poorly or which route poorly to lysosomes.

### SUMMARY OF THE INVENTION

In a first aspect, the present invention relates to a multispecific antigen-binding molecule comprising a first antigen-binding domain and a second antigen-binding domain, wherein the first domain specifically binds a target molecule (T), and wherein the second domain specifically binds an internalizing effector protein (E), and wherein the second antigen-binding domain has a dissociation constant K_{D} with E of between 10⁻⁹ and 10⁻⁸ M.

It has been found by the present inventors that the specific affinity range of the second antigen-binding domain bestows surprisingly beneficial properties on the multispecific antigen-binding molecule of the present invention. The second antigen-binding domain is found within a specific range of binding affinities to readily induce internalization of the multispecific molecule as well as to induce cytotoxicity of the drug-conjugated multispecific molecule. This applies in particular where the first domain specifically binds a tumor-associated antigen such as for example HER2. At the same time, the second antigen-binding domain exerts only limited internalization and cytotoxicity (when employed in the context of an ADC) within the specific range of binding affinities, without binding of the first binding domain, i.e. demonstrating surprisingly low cytotoxicity in cells that express the internalizing effector protein (E) in the absence of the tumor-associated target (T). Thus, the multispecific antigen-binding molecule of the present invention demonstrates binding, internalization, lysosomal routing and toxin release in tumor cells, accompanied by minimal internalization, lysosomal routing and toxin release in non-tumor cells.

It has also been found by the present inventors that the multispecific antigen-binding molecule of the present invention allows for utilization of tumor antigens that usually do not internalize or poorly internalize, thereby greatly enhancing the pool of potential ADC targets.

In another aspect, the present invention relates to the multispecific antigen-binding molecule, wherein the molecule is conjugated to a cytotoxic moiety, a radioisotope, a drug, a cytokine, or an RNA silencing vehicle. In other aspects, the present invention relates to the use of the multispecific antigen-binding molecule in a method for treating and/or preventing a cancer, and to the use of the multispecific antigen-binding molecule in a method of targeting a tumor in a subject, the method comprising administering to the subject the multispecific antibody or ADC.

In other aspects, the present invention relates to a pharmaceutical composition comprising the multispecific antigen-binding molecule as an active ingredient, to nucleic acid(s) encoding the multispecific antigen-binding molecule, to an expression vector containing said nucleic acid(s) and being capable of expressing said nucleic acids in a single or multiple prokaryotic or eukaryotic host cel lines as appropriate, and to prokaryotic or eukaryotic host cell lines comprising said vector(s).

### DEFINITIONS

### Binding, affinity, and K_{D}

The term "binding" as used herein refers to the binding of an antigen-binding molecule such as an antibody to a predetermined antigen or target, e.g. with a binding affinity corresponding to a K_{D} value of about 10⁻⁸ M or less.

The skilled reader will be familiar with the concept of affinity and the equilibrium dissociation constant K_{D}. The dissociation constant K_{D} can be measured by biolayer.

K_{D} values may be determined by biolayer interferometry (BLI) in an Octet HTX instrument using the antigen-binding molecule, e.g. the antibody, as the immobilized ligand and the antigen as the analyte.

K_{D} (M) refers to the dissociation equilibrium constant of a particular interaction between a multispecific-antigen binding molecule and an antigen, preferably the interaction between a single binding arm of an antibody molecule and an antigen, and may be obtained by dividing k_{d} by kₐ. The term "k_{d}" (sec⁻¹), as used herein, refers to the dissociation rate constant of a particular interaction between a multispecific-antigen binding molecule and an antigen. Said value is also referred to as the k_{dis}, k_{off} value or off-rate. The term "kₐ" (M⁻¹ x sec⁻¹), as used herein, refers to the association rate constant of a particular interaction between a multispecific-antigen binding molecule and an antigen. Said value is also referred to as the kₒₙ value or on-rate.

### Antibody

As used herein, the term "antibody" (Ab) in the context of the present invention refers to an immunoglobulin molecule, a fragment of an immunoglobulin molecule, or a derivative of either thereof, which has the ability to specifically bind to an antigen, preferably dual binding to two different antigens such as for bispecific antibodies, under typical physiological conditions for a relevant functionally-defined period to induce, promote, enhance, and/or modulate a physiological response associated with antibody binding to the antigen.

The variable regions of the immunoglobulin molecule (either of the heavy and/or light chains or heavy chains only) contain a binding domain that interacts with an antigen. The constant regions of the antibodies (Abs) may mediate the binding of the immunoglobulin to FcRn. An antibody may also be a bispecific or multispecific antibody, such as but not limited to: DuoBody molecules, tandem scFv, tandem scFv-Fc, knob-into-hole IgGs, scFv-Fc knobs-into-holes, scFv-Fc-scFv, F(ab')2, Fab-scFv, (Fab'scFv)2, Diabody, scDiabody, scDiabody-Fc, or scDiabody-CH3, Triomab, kih IgG common LC, CrossMab, DVD-Ig, 2 in 1-IgG, IgG-scFv, bi-Nanobody, BiTE, TandAbs, DART, DART-Fc, scFv-HSA-scFv, orthoFab-IgG, tetravalent Tv-IgGs, dock-and-lock (DNL) formats such as DNL-Fab3 and Azymetric scaffold, or similar molecule.

As indicated above, the term antibody herein, unless otherwise stated or clearly contradicted by context, includes fragments of an antibody that are antigen-binding fragments, i.e., retain the ability to specifically bind to the antigen. It has been shown that the antigen-binding function of an antibody may be performed by fragments of a full-length antibody. Examples of antigen-binding fragments encompassed within the term "antibody" include (i) a Fab' or Fab fragment, a monovalent fragment consisting of the VL, VH, CL and CH1 domains, or a monovalent antibody as described in WO2007059782 (Genmab) ; (ii) F(ab')2 fragments, bivalent fragments comprising two Fab fragments linked by a disulfide bridge at the hinge region; (iii) a Fd fragment consisting essentially of the VH and CH1 domains; (iv) a Fv fragment consisting essentially of the VL and VH domains of a single domain of an antibody, (v) a dAb fragment, which consists essentially of a VH domain and also called domain antibodies; (vi) camelid or nanobodies and (vii) an isolated complementarity determining region (CDR). Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv)). Such single chain antibodies are encompassed within the term antibody unless otherwise noted or clearly indicated by context. These and other useful antibody fragments in the context of the present invention, as well as bispecific formats of such fragments, are discussed further herein. It also should be understood that the term antibody, unless specified otherwise, also includes polyclonal antibodies, monoclonal antibodies (mAbs), antibody-like polypeptides, chimeric antibodies, humanized and fully human antibodies, and antibody fragments retaining the ability to specifically bind to the antigen (antigen-binding fragments) provided by any known technique, such as enzymatic cleavage, peptide synthesis, and recombinant techniques. An antibody as generated can possess any isotype.

As used herein, "isotype" refers to the immunoglobulin (sub)class (for instance IgG1, IgG2, IgG3, IgG4, IgD, IgA, IgE, or IgM) that is encoded by heavy chain constant region genes.

The term "monovalent antibody" means in the context of the present invention that an antibody molecule is capable of binding no more than a single molecule of the antigen.

The term "bivalent antibody" means in the context of the present invention that the antibody molecule contains two binding domains for a specific antigen, and is therefore capable of binding one or two molecules of that antigen

### Generation of multispecific antibodies

The multispecific antibodies, in particular the bispecific antibodies, of the present invention may be generated through controlled Fab-arm exchanged (FAE) as described in Labrijn et al., Efficient generation of stable bispecific IgG1 by controlled Fab-arm exchange, PNAS, vol. 110, no. 13, pp. 5145-5150, March 2013*,* in WO 2011/131746 A2, or in Labrijn et al. Controlled Fab-arm exchange for the generation of stable bispecific IgG1, Nat Protoc, 2014 Oct;9(10):2450-63, also known as DuoBody^{®} technology. Briefly, in this in vitro method, two different antibodies are provided, both comprising an Fc region of an immunoglobulin with a CH3 region, wherein the sequences of the respective CH3 regions are different and contain a matched mutation.

As a result, the heterodimeric interaction between the first and second CH3 regions is stronger than each of the homodimeric interactions of said first and second CH3 regions. The antibodies are incubated under reducing conditions to allow the cysteines in the hinge region to undergo disulfide-bond isomerization, and to obtain the bispecific antibody by controlled Fab-arm exchange. The reducing agent is thereupon removed from the mixtures (now containing bispecific antibodies) to allow oxidation of the disulfide bonds. The sequences of said CH3 regions contain matched mutations, i.e. mutations at different positions in the two CH3 regions, preferably a mutation at position 405 in one of the CH3 regions of an IgG1 molecule and a mutation at position 409 in the CH3 region of another IgG1 molecule. Multispecific antibodies may also be generated using other technologies and formats, such as but not limited to: tandem scFv, tandem scFv-Fc, knob-into-hole IgGs, scFv-Fc knobs-into-holes, scFv-Fc-scFv, F(ab')2, Fab-scFv, (Fab'scFv)2, Diabody, scDiabody, scDiabody-Fc, or scDiabody-CH3, Triomab, kih IgG common LC, CrossMab, DVD-Ig, 2 in 1-IgG, IgG-scFv, bi-Nanobody, BiTE, TandAbs, DART, DART-Fc, scFv-HSA-scFv, orthoFab-IgG, tetravalent Tv-IgGs, dock-and-lock (DNL) formats such as DNL-Fab3 and Azymetric scaffold, or similar molecule.

### Induction of internalization of a multispecific antigen-binding molecule

Binding of both T and E by the multispecific antigen-binding molecule preferably induces internalization of the multispecific antigen-binding molecule of the present invention, as well as cytotoxicity of a multispecific drug-conjugated antibody of the present invention, to a greater extent than by binding to target T alone. For instance, internalization induced by binding, preferably simultaneous binding, of T and E by the multispecific antigen-binding molecule may be more than 10%, such as more than 20%, more than 30%, more than 40%, more than 50%, more than 60%, more than 70%, more than 80%, more than 90%, more than 100%, more than 110%, more than 150%, more than 200%, more than 300%, more than 400%, more than 500%, more than 750%, more than 1000%, more than 2000%, or more than 5000% higher than the level of internalization measured in the presence of a control construct containing only binding to T and not to E.

A non-limiting example of determining whether the multispecific antigen-binding molecule of the present invention enhances internalization of the multispecific antigen-binding molecule to a greater extent than the binding of the target molecule (T) by the first domain alone is shown in the co-localization assays in Examples 5 and 8, or in the HER2 downmodulation assay of Example 9, as discussed below. In those examples, T is HER2 and E is CD63. As to the concept of enhanced internalization, in Example 13 and 14, enhanced internalization of multispecific antibodies is demonstrated using Integrin beta-1 (CD29) as the target (T) and CD63 as the internalizing effector protein (E) that is inducing enhanced internalization.

The present inventors have found that, in particular when E is CD63, the specific affinity range of the second antigen-binding domain bestows surprisingly beneficial properties on the bispecific antigen-binding molecule of the present invention. Monovalent binding of the antigen-binding domain to E within specific affinity range of a dissociation constant K_{D} 10⁻⁹ to 10⁻⁸ M readily induces internalization of bispecific antibodies and cytotoxicity of bispecific drug-conjugated antibodies, in particular when the first domain specifically binds a tumor-associated antigen such as e.g. HER2. Moreover, when only E and not the tumor-associated target antigen (T) is present, the second antigen-binding domain exerts only limited contribution to internalization of the multispecific antigen-binding molecule and cytotoxicity of multispecific drug-conjugated antibodies within this specific affinity range, i.e. demonstrating surprisingly low or absent cytotoxicity in cells that express the internalizing effector protein (E) in absence of the tumor target (T). Thus, the multispecific antigen-binding molecule of the present invention demonstrates binding, internalization, lysosomal accumulation in tumor cells, accompanied by minimal internalization into non-tumor cells.

### CD63

The Cluster of Differentiation 63 (CD63, Uniprot ID P08962) molecule is also known as lysosome-associated membrane glycoprotein 3 (LAMP-3). CD63 is also known as: platelet glycoprotein 40 (Pltgp40), melanoma antigen ME491 or MLA1, ocular melanoma-associated antigen (OMA81H), tetraspanin-30 (TSPAN30), granulophysin or lysosomal integral membrane protein-1 (LIMP-1). CD63 is a member of the tetraspanin superfamily and is ubiquitously expressed. The CD63 gene is located on human chromosome 12q13 and was the first characterized tetraspanin. Originally, CD63 was discovered as a protein present on the cell surface of activated blood platelets, known as Pltgp40 and in early stage human melanoma cells, where it was known as ME491.

CD63 is expressed in many cell types. Amongst others, CD63 is expressed intracellularly in lysosomes, endosomes, granules of resting platelets and basophils. Cell surface expression of CD63 can be detected on activated basophils and platelets, monocytes, macrophages, and granulocytes. CD63 is also expressed on endothelial cells, fibroblasts, osteoblasts, neural tissue, melanoma cells, smooth muscle cells and mast cells. CD63 is described to shuttle between the plasma membrane and intracellular compartments.

The major pool of CD63 resides in intracellular compartments such as endosomes and lysosomes, but some expression can be found on the cell surface. CD63 has been described to regulate transport of other proteins typically through endocytosis. Furthermore, CD63 has been described to regulate surface expression of membrane-type 1 matrix metalloproteinase by targeting the enzyme for lysosomal degradation, and silencing of CD63 in endothelial cells prevents internalization of vascular endothelial growth factor receptor 2 (VEGFR2) in response to its ligand VEGF. Also across different tumor types, CD63 has been demonstrated to continuously shuttle between the plasma membrane and lysosomes, which was dependent on the presence of AP2 and clathrin. Thus CD63 seems an attractive antigen to facilitate internalization and lysosomal delivery, a feature suitable for enhancing efficacy of certain antibody drug conjugates (ADC) targeting tumor antigens that by themselves to not internalize and/or shuttle to lysosomes sufficiently.

CD63 expression does not show a tumor-specific expression, although CD63 was first discovered as an abundantly expressed surface antigen in early stage melanoma cells. CD63 cell surface expression is reduced during malignant melanoma progression, indicative of a negative correlation between cell surface expression of CD63 and tumor invasiveness.

### Tumor-associated antigens

Tumor-associated antigens are antigens that are expressed on the surface of (certain) tumor cells, and for which surface expression to a lesser extent is found on normal cells. As used herein, the term "tumor-associated antigen" refers to proteins or polypeptides, but also carbohydrates, glycoproteins, lipids, lipoproteins, lipopolysaccharides, or other non-protein polymers that are preferentially expressed on the (outside) surface of a tumor cell. The term "preferentially expressed," as used in this context, means that the antigen is expressed on a tumor cell at a level that is at least 10%, such as at least 20%, at least 30%, at least 40%, at least 50%, at least 60%, at least 70%, at least 80%, at least 90%, at least 100%, at least 110%, at least 150%, at least 200%, at least 300%, at least 400%, at least 500%, at least 750%, at least 1000%, at least 2000% or at least 5000% greater than the expression level of the antigen on non-tumor cells. Tumor-associated antigens can derive from any protein, glycoprotein or other macromolecules synthesized by the tumor cell.

Tumor-associated antigens may be grouped in different classes of antigens: 1) Class I HLA-restricted cancer testis antigens which are expressed normally in the testis or in some tumors but not in normal tissues, including antigens from the MAGE, BAGE, GAGE, NY-ESO and BORIS families; 2) Class I HLA restricted differentiation antigens, including melanocyte differentiation antigens such as MART-1, gp100, PSA, Tyrosinase, TRP-1 and TRP-2; 3) Widely expressed antigens, which are antigens expressed both in normal and tumor tissue though at different levels or altered translation products, including CEA, HER2/neu, hTERT, MUC1, MUC2 and WT1; 4) Tumor specific antigens which are unique antigens that arise from mutations of normal genes including β-catenin, α-fetoprotein, MUM, RAGE, SART, etc; 5) Viral antigens such as HPV, EBV; and 6) Fusion proteins, which are proteins created by chromosomal rearrangements such as deletions, translocations, inversions or duplications that result in a new protein expressed exclusively by the tumor cells, such as Bcr-Abl.

Preferred examples of tumor-associated antigens include 5T4, A33, activin receptor, adrenomedullin receptors, AFP, AGS-5, ALK, annexin, AXL, B7-H3, B7-H4, BAGE proteins, BCMA, Bombesin, C33 antigen, C4.4a, C-type lectin-like(-receptor), CA19.9, CA-125, CADM1, CAIX, CanAg, CAR, carbonic anhydrase, Caveolin-1, CCK2R, CD4, CD10, CD19, CD20, CD21, CD22, CD25, CD27, CD30, CD33, CD37, CD38, CD44, CD51, CD57, CD70, CD73, CD74, CD79a, CD79b, CD80, CEA, CEACAMs, c-kit, claudin, chemokine receptors (i.e., CXCR4, CXCR5), c-Met, Cripto-1, DEC-205, Derlin-1, Desmoglein-3, Dlk-1, DLL3, DS6, E-cadherin, E-Selectin, EAG-1, ED-B, EpCAM, EGFR, EGFRvIII, emmprin, endothelin receptor, ErbB2/Her2, ErbB3, ErbB4, ETV6-AML, Ephrin type-A receptor, Epiregulin, ETA, FAP-alpha, FcyR's, FGFR, FOLR1, Frizzled, Fyn3, Galectin, Ganglioside, GCC, GD2, GD3, GloboH, lypican-3, GLUT3, GPNMB, G-protein coupled receptors (i.e., GPR49), gp100, Hsp, HLA/B-raf, HLA-DR, HLA/k-ras, HLA MAG E-A3, HMW-MAA, hTERT, ICAM-3, IGF-R, IL-13-R, L1CAM, laminin receptor, LIV1, LMP2, LRP5, LRP6, MAGE proteins, MART-1, melanotransferrin, mesothelin, metalloproteinase, ML-IAP, Mucins, Mud, Mud 6 (CA-125), MU M1, N-cadherin, NA17, NCAM-1, Nectin4, Notch, NP-55, NRP1, NY-BR1, NY-BR62, NY-BR85, NY-ES01, PLAC1, PRLR, PRAME, prominin-1, PSMA (FOLH 1 ), RON, SLC44A4, SLITRK6, Steap-1, Steap-2, surviving, syndecan, TAG-72, TF, TGF-β, TMPRSS2, TMEFF2, TNFR, Tn, TROP2, TRP-1, TRP-2,TWEAKR, tyrosinase, uroplakin-3 and VEGFR.

Preferred examples of tumor-associated antigens include tumor-associated antigens that are highly overexpressed, but lack sufficient lysosomal transport, such as glycosylphosphatidylinositol (GPI) anchored proteins (i.e., glypican family, uPAR, folate binding receptors, prostasin, FcgRlllb [CD16b], alkaline phosphatase, acetylcholinesterase, 5' nucleotidase [p36], Cripto, LFA-3 [CD58], DAF [CD55], Thy-1 [CD90], Qa-2, Ly-6A and MIRL [CD59]), adhesion molecules (i.e., selectins, L1CAM, N-CAM, LRP1, TAG1, cadherins), which are often recycle back to the plasma membrane after endocytosis, with only a minor fraction being targeted for lysosomal degradation

Preferred examples of tumor-associated antigens include tumor-specific antigens that are known to interact with CD63 which may improve bivalent binding of bsADC at low copy numbers. For example, CD63 has been described to interact with other tetraspanins (i.e., CD81, CD82, CD9 and CD151), integrins, MHCII, CXCR4, TM4SF5, syntenin-1, TIMP-1, H, K-ATPase, L6-antigen and MT1-MMP.

Examples of tumor-associated antigens include selection of glycotargets such as, Lewis-Y (CD174), Lewis-X (CD15), SLe^{X}, SLe^{A}, sTn, fucosyl-GM1, Globo H, SSEA-3, GM2, GD2, GD3, Polysialic acids or glycoproteins (i.e., Mucins).

### DESCRIPTION OF THE FIGURES

Figure 1 shows a dose response curve of anti-CD63 antibodies binding to recombinant human CD63 as measured by ELISA.
Figure 2 shows affinity measurements of affinity variants of anti-CD63 antibodies measured with label-free Bio-Layer Interferometry.
Figure 3 shows the results of a viability assay to test the cytotoxicity of monovalent bsCD63_{N74H}xb12-Duo3 ADCs and bsHER2xCD63_{n74H}-Duo3 ADCs in Colo205 cells.
Figure 4 shows the results of a viability assay to test the cytotoxicity of monovalent bsCD63_{N74H}xb12-Duo3 ADCs and bsHER2xCD63_{N74H}-Duo3 ADCs in SK-OV-3 cells.
Figure 5 shows the results of a viability assay to test the cytotoxicity of monovalent bsCD63_{N74H}xb12-Duo3 ADCs and bsHER2xCD63_{N74H}-Duo3 ADCs in HCC1954 cells.
Figure 6 shows lysosomal co-localization of monovalent bsCD63_{N74H}xb12 bispecific molecules measured in SK-OV-3 cells with confocal microscopy.
Figure 7 shows binding of bsHER2xCD63_{N74H} to SK-OV-3 cells as determined by flow cytometry.
Figure 8 shows intracellular accumulation of FITC-conjugated CD63 antibody and CD63 affinity variant antibodies in granulocytes and thrombocytes.
Figure 9 shows lysosomal co-localization of bsHER2xCD63_{N74H} in SK-OV-3 cells followed over time.
Figure 10 shows the total amount of HER2 protein in tumor cell lines with different expression levels of HER2, as quantified by ELISA after three days of incubation with bsHER2xCD63_{N74H},compared with untreated cells.
Figure 11 shows the results of a viability assay to test the cytotoxicity of Duostatin-3 conjugated bispecific ADCs in vitro.
Figure 12 shows the mean tumor size and percentage of tumor free survival in mice that had been subcutaneously inoculated with SK-OV-3 tumor xenografts, followed by treatment withDuostatin-3 conjugated multispecific ADCs.
Figure 13 shows binding of bsBeta1xCD63_{N74H} to SK-OV-3 cells as assessed by flow cytometry.
Figure 14 shows lysosomal co-localization of bsBeta1xCD63_{N74H} on SK-OV-3 cells.
Figure 15 shows lysosomal co-localization of bsBeta1xCD63_{N74H} on SK-OV-3 cells followed over time.

### DETAILED DESCRIPTION OF THE INVENTION

In one aspect, the present invention relates to a multispecific antigen-binding molecule comprising a first antigen-binding domain and a second antigen-binding domain, wherein the first domain specifically binds a target molecule (T), and wherein the second domain specifically binds an internalizing effector protein (E), and wherein the second antigen-binding domain has a dissociation constant K_{D} value with E of between 10⁻⁹ and 10⁻⁸ M.

To provide tumor specificity of the multispecific antigen-binding molecule of the present invention, in absence of the first antigen-binding domain, the second domain which specifically binds to the internalizing effector protein (E), may advantageously not bind or only bind with low affinity and subsequently not internalize or at least internalize to a significant lesser degree. It has been found by the present inventors that a multispecific antigen-binding molecule wherein the second antigen-binding domain has a dissociation constant K_{D} value with E of between 10⁻⁹ and 10⁻⁸ M fulfills these criteria.

The target molecule (T), preferably a tumor-associated target molecule, may be a protein, polypeptide, lipid or other macromolecule. In a preferred embodiment, the target molecule is a protein. In another embodiment, the target molecule, preferably a tumor-associated target molecule is a polypeptide. In some embodiments, T is a cell surface-expressed target protein or target polypeptide. In other embodiments, T is a soluble target protein or target polypeptide, preferably one that interacts with a cell surface receptor. Target binding by the multispecific antigen-binding molecule may take place extracellularly or on the cell surface.

In one embodiment, the target molecule is a cell surface-expressed receptor. In a preferred embodiment, the target molecule is a tyrosine kinase receptor, preferably a transmembrane tyrosine kinase receptor. In another embodiment, the target molecule is a membrane-bound ligand.

In other embodiments, the multispecific antigen-binding molecule, preferably bispecific antigen-binding molecule, binds E on the cell surface or inside the cell.

It is particularly preferred that the target molecule is a tumor-associated antigen, such as a tumor-associated protein or polypeptide. Advantageously, the tumor-associated antigen is an antigen that is not ordinarily internalized or is poorly internalized. Preferably, the tumor-associated antigen is an antigen that shows inefficient routing to the lysosomal compartment.

The internalizing effector protein (E) may be tumor-associated or tumor-specific. In other embodiments, the internalizing effector protein (E) may be expressed on or in tumor as well as non-tumor cells.

The internalizing effector protein (E) is a protein that is capable of being internalized into a cell or that otherwise participates in or contributes to internalization. In some embodiments, the internalizing effector protein is a protein that undergoes transcytosis; i.e. the protein is internalized on one side of a cell and transported to the other side of the cell. Preferably, the internalizing effector protein is a membrane protein or a soluble extracellular protein that binds a membrane-bound receptor. In a preferred embodiment, the internalizing effector protein is a protein that shows efficient routing to the lysosomal compartment of the cell.

Binding of the second domain to the internalizing effector protein advantageously results in internalization of the multispecific antigen-binding molecule and the target molecule associated therewith into the cell. In a preferred embodiment, the internalizing effector protein is a membrane-associated protein with at least one extracellular domain or region, the protein being internalized, and preferably processed via an intracellular degradative and/or recycling pathway. Specific examples of internalizing effector proteins that are directly internalized into a cell include, e.g., CD63, MHC-I (e.g., HLA-B27), Kremen-1 , Kremen-2, LRP5, LRP6, LRP8, transferrin receptor, LDL-receptor, LDL-related protein 1 receptor, ASGR1 , ASGR2, amyloid precursor protein-like protein-2 (APLP2), apelin receptor (APLNR), MAL (Myelin And Lymphocyte protein, VIP17), IGF2R, vacuolar-type H+ ATPase, diphtheria toxin receptor, folate receptor, glutamate receptors, glutathione receptor, leptin receptors, scavenger receptors (e.g., SCARA1-5, SCARB1-3, CD36). In a preferred embodiment, the internalizing effector protein E is a cell surface internalizing receptor. In a preferred embodiment, the internalizing effector protein E is CD63.

In a preferred embodiment, the multispecific antigen-binding molecule comprises i) a first binding arm which comprises the first antigen-binding domain and ii) a second binding arm which comprises the second antigen-binding domain.

It is particularly preferred that the multispecific antigen-binding molecule is a bispecific antigen-binding molecule.

In one embodiment of the present invention, the second antigen-binding domain has a dissociation constant K_{D} value with E higher than 10⁻⁹ and lower than 10⁻⁸ M. According to another embodiment, the second antigen-binding domain has a dissociation constant K_{D} with E of between 2.0x10⁻⁹ and 9.0x10⁻⁹ M. According to another embodiment, the second antigen-binding domain has a dissociation constant K_{D} with E of between 2.0x10⁻⁹ and 7.3x10⁻⁹ M.

According to another embodiment, E is a cell surface-expressed molecule that is internalized, preferably directly internalized, into the cell. Preferably, the multispecific antigen-binding molecule is internalized into the cell by way of binding to E only in the presence of the target molecule (T). It is also preferred that the multispecific antigen-binding molecule is internalized into the cell by way of binding to E only when the first domain is specifically bound to the target molecule (T).

In one embodiment, the multispecific antigen-binding molecule, upon binding to E, internalizes more efficiently into cells expressing T as compared to cells not expressing T.

In another embodiment, the multispecific antigen-binding molecule, upon binding to T, internalizes more efficiently into cells expressing E as compared to cells not expressing E.

In another embodiment, the multispecific antigen-binding molecule, upon binding to E, is transported to the lysosomal compartment in cells expressing T.

In another embodiment, the multispecific antigen-binding molecule, upon binding to E, is more efficiently transported to the lysosomal compartment in cells expressing T as compared to cells not expressing T.

In another embodiment, the multispecific antigen-binding molecule, upon binding to T, is more efficiently transported to the lysosomal compartment in cells expressing E as compared to cells not expressing E.

According to another embodiment, E is selected from the group consisting of CD63, MHC-I, Kremen-1, Kremen-2, LRP5, LRP6, transferrin receptor, LDLr, MAL, V-ATPase and ASGR. In a preferred embodiment, E is CD63.

According to another embodiment, E is a soluble ligand that is internalized into a cell via the interaction between E and an internalizing cell surface-expressed receptor molecule.

According to another embodiment, T is a cell surface-expressed target molecule. In a particularly preferred embodiment, T is a tumor-associated antigen. The internalization-enhancing strategy of the present invention may thus involve combining a tumor-associated target antigen with the internalizing capacities of an antigen such as CD63. In particular, bispecific antibodies that bind to both CD63 and a tumor-associated target may be useful in therapeutic settings in which specific targeting and enhanced internalization of an antibody-drug-conjugate is desired. According to one embodiment, T is HER2.

According to another embodiment, the first and/or the second antigen-binding domain comprises at least one antibody variable region, preferably at least two antibody variable regions.

According to some embodiments, the multispecific antigen-binding molecule is a multispecific antibody, preferably a bispecific antibody, or a multispecific, preferably bispecific, antibody fragment or recombinantly engineered part thereof. In a particularly preferred embodiment, the multispecific antigen-binding molecule is a bispecific antibody. A bispecific antibody may be employed to use internalization enhancing properties of one antigen by binding to the same with one arm, and bind a target molecule, such as a tumor-associated target molecule, with the other arm of the bispecific antibody. Such a bispecific antibody may then be loaded with a cytotoxic conjugate to induce cell death upon internalization of the ADC.

In one embodiment, the antibody is a bispecific antibody, comprising (i) a first antibody comprising a first antigen-binding domain specifically binding a target molecule (T) as defined herein, and (ii) a second antibody comprising a second antigen-binding domain specifically binding an internalizing effector protein (E) as defined herein.

In a preferred embodiment, the multispecific antigen-binding molecule is a bispecific antibody comprising a first binding arm comprising the first antigen-binding domain and a second binding arm comprising said second antigen-binding domain. Advantageously, said first antigen-binding domain comprises a first heavy chain variable sequence (VH) and a first light chain variable sequence (VL), and said second antigen-binding domain comprises a second heavy chain variable sequence (VH) and a second light chain variable sequence (VL) and wherein said variable sequences each comprises three CDR sequences, CDR1, CDR2 and CDR3.

In a preferred embodiment, (i) said first binding arm comprises a first heavy chain comprising a first heavy chain variable sequence (VH) and a first heavy chain constant sequence (CH), and a first light chain comprising a first light chain variable sequence (VL) and a first light chain constant sequence (CL), and (ii) said second binding arm comprises a second heavy chain comprising a second heavy chain variable sequence (VH) and a second heavy chain constant sequence (CH), and a second light chain comprising a second light chain variable sequence (VL) and a second light chain constant sequence (CL).

According to another embodiment, the first binding arm is derived from a chimeric antibody or from a humanized antibody or from a human antibody. According to another embodiment, the second binding arm is derived from a chimeric antibody or from a humanized antibody or from a human antibody. Accordingly, in one embodiment the first binding arm is derived from a human antibody and the second binding arm is derived from a humanized antibody or from a chimeric antibody.

It is preferred that the multispecific antigen-binding molecule of the present invention is a bispecific antibody, wherein the bispecific antibody is a full-length antibody, preferably an IgG1 antibody.

In a preferred embodiment, the multispecific antigen-binding molecule of the invention is isolated. An "isolated multispecific antigen-binding molecule" as used herein, is intended to refer to a multispecific antigen-binding molecule, such as a bispecific antibody, which is substantially free of other antigen-binding molecules or antibodies having different antigenic specificities. Moreover, an isolated multispecific antigen-binding molecule may be substantially free of other cellular material and/or chemicals.

According to another embodiment, said second antigen-binding domain has one or more mutations that modulate the affinity of the second antigen-binding domain with E. In another embodiment, said second antigen-binding domain is derived from an antibody having one or more mutations in the VH and/or VL that modulates the affinity of the second antigen-binding domain with E. It is preferred that the affinity is modulated such that the second antigen-binding domain has a dissociation constant K_{D} value with E of between 10⁻⁹ and 10⁻⁸ M.

According to another embodiment, said antibody has one or more mutations in the anti-CD63 Fab region that modulates the affinity of the second antigen-binding domain with E, where E is CD63. According to another embodiment, the mutation is a single amino acid substitution, preferably a single amino acid histidine substitution.

According to another embodiment, said one or more mutations in the VH and/or VL is an amino acid substitution, preferably a histidine substitution, at position 54 of the VL according to SEQ ID No. 5 of Table 1 below, or at positions 71, 72 and/or 74 of the VH according to SEQ ID No. 1 of Table 1 below. For example, anti-CD63-N74H has an asparagine to histidine mutation at position 74 of the heavy chain according to SEQ ID No. 1, anti-CD63-LN54H has an asparagine to histidine mutation at position 54 of the light chain according to SEQ ID No. 5. According to another embodiment, the second antigen-binding domain is selected such that it binds target E with a K_{D} within the preferred affinity range.

In a preferred embodiment, said amino acid substitution, preferably a histidine substitution is at position 74 of the VH according to SEQ ID No. 1. In a preferred embodiment, the mutation is N74H of the VH according to SEQ ID No. 1.

In a preferred embodiment said second domain, preferably as part of said second binding arm, comprises:
a) VH CDRs 1, 2, and 3 as provided in SEQ ID Nos: 2, 3, and 4, respectively, and VL CDRs 1, 2, and 3 as provided in SEQ ID Nos: 9, 7, and 8, respectively, or
b) VH CDRs 1, 2, and 3 as provided in SEQ ID Nos: 2, 10, and 4, respectively, and VL CDRs 1, 2, and 3 as provided in SEQ ID Nos: 6, 7, and 8, respectively, or
c) VH CDRs 1, 2, and 3 as provided in SEQ ID Nos: 2, 11, and 4, respectively, and VL CDRs 1, 2, and 3 as provided in SEQ ID Nos: 6, 7, and 8, respectively, or
d) VH CDRs 1, 2, and 3 as provided in SEQ ID Nos: 2, 12, and 4, respectively, and VL CDRs 1, 2, and 3 as provided in SEQ ID Nos: 6, 7, and 8, respectively.

In a particularly preferred embodiment, said second domain, preferably as part of said second binding domain, comprises VH CDRs 1, 2, and 3 as provided in SEQ ID Nos: 2, 12, and 4, respectively, and VL CDRs 1, 2, and 3 as provided in SEQ ID Nos: 6, 7, and 8, respectively.

According to another embodiment, said multispecific antigen-binding molecule comprises a mutated Fab region of a CD63-specific monoclonal antibody. According to another embodiment, said multispecific antigen-binding molecule comprises a mutated Fab region of CD63-specific monoclonal Ab 2192. According to another embodiment, said multispecific antigen-binding molecule comprises an antigen-binding region specific for CD63 selected from a hybridoma or phage-display library.

According to another embodiment, said first and second antigen-binding domains are each a pair of an antibody heavy chain variable domain and an antibody light chain variable domain.

The bispecific antigen-binding molecule may preferably further comprise antibody constant regions.

According to another embodiment, the antigen-binding molecule is a tumor-associated target (T)xCD63 bispecific antibody. In a preferred embodiment, the (T)xCD63 bispecific antibody is conjugated to a cytotoxic drug. In one embodiment the (T)xCD63 bispecific antibody is conjugated to duostatin-3. In one embodiment the (T)xCD63 bispecific antibody is conjugated to duostatin-3 and the anti-CD63 binding domain, which is the second binding domain, comprises VH CDRs 1, 2, and 3 as provided in SEQ ID Nos: 2, 12, and 4, respectively, and VL CDRs 1, 2, and 3 as provided in SEQ ID Nos: 6, 7, and 8, respectively. In particular, tumor-associated target (T)xCD63 bispecific antibody-drug conjugates (ADC) are useful in therapeutic settings in which specific targeting and enhanced internalization of the antibody-drug-conjugate is desired. It has been found by the inventors that the (T)xCD63 bispecific ADCs of the present invention are more efficient in killing cells expressing target T when compared to targeting only the tumor-associated antigen using a monospecific ADC. Such ADCs are found to be more potent in eradicating tumor cells in vitro and in animal models than prior art bispecific ADCs.

The (T)xCD63 bispecific ADCs of the present invention are found to be advantageous by enhancing the internalization of the ADC by being able to bind both the tumor-associated target and the potent internalizing characteristics of the CD63 antigen, thereby inducing stronger killing of cells by more efficient payload delivery inside the targeted cells. To tailor to the need of increasing efficacy of antibodies targeting various tumor-antigens, a narrow range of surprisingly efficient CD63 affinity variants (spanning a range of CD63 affinities) was found to enhance efficacy of the bispecific ADCs.

According to another embodiment, the antigen-binding molecule is a HER2xCD63 bispecific antibody.

According to another embodiment, the antigen-binding molecule has an EC₅₀ value for binding to tumor-associated target (T)-expressing cells, such as HER2-expressing cells, of lower than 5.0 µg/ml, such as lower than 4.0 µg/ml, such as lower than 3.0 µg/ml, such as lower than 2.0 µg/ml, such as lower than 1.0 µg/ml, such as lower than 0.9 µg/ml, such as lower than 0.8 µg/ml, such as lower than 0.7 µg/ml, such as lower than 0.6 µg/ml, such as lower than 0.5 µg/ml, such as lower than 0.4 µg/ml, such as lower than 0.3 µg/ml, such as lower than 0.2 µg/ml, such as lower than 0.1 µg/ml, such as lower than 0.05 µg/ml, such as lower than 0.01 µg/ml, as determined by flow cytometry.

In another preferred embodiment, the binding of T and E by the multispecific antigen-binding molecule induces internalization of the multispecific antigen-binding molecule to a greater extent than the binding of T by the first domain alone. Similarly, the binding of T and E by the multispecific drug-conjugated antibodies of the present invention preferably induces cytotoxicity to a greater extent than the binding of T by the first domain alone.

In another preferred embodiment, the binding of T and E by the multispecific, preferably bispecific, antigen-binding molecule induces internalization of the multispecific antigen-binding molecule to a greater extent than the corresponding bivalent monospecific antibody binding T. Similarly, the binding of T and E by the multispecific, preferably bispecific, drug-conjugated antibodies of the present invention preferably induces cytotoxicity to a greater extent than the than the corresponding bivalent monospecific ADC binding T.

According to another embodiment, the K_{D} value is determined by biolayer interferometry at 30 °C. In another embodiment, K_{D} is determined by biolayer interferometry at 30 °C and a pH of between 7.2 and 7.5, such as between 7.3 and 7.4, such as pH 7.4. In another embodiment, K_{D} is determined by biolayer interferometry at 30 °C at 1000 RPM shaker speed. In another embodiment, K_{D} is determined by biolayer interferometry using an Octet system, such as Octet HTX (ForteBio).

In a preferred embodiment, the antigen-binding molecule is a bispecific antibody comprising:
i) a first binding arm comprising a first heavy chain comprising a first heavy chain constant sequence (CH), said first CH comprising a first CH3 region, and
ii) a second binding arm comprising a second heavy chain comprising a second heavy chain constant sequence (CH), said second CH comprising a second CH3 region,
wherein the sequences of said first and second CH3 regions are different and are such that a heterodimeric interaction between said first and second binding arm is stronger than a homodimeric interaction of each of said first and second binding arms.

Preferably, in said first heavy chain CH3 region at least one of the amino acids in a position corresponding to positions T366, L368, K370, D399, F405, Y407 or K409 of human IgG1 heavy chain has been substituted, and in said second heavy chain CH3 region at least one of the amino acids in a position corresponding to positions T366, L368, K370, D399, F405, Y407 or K409 of human IgG1 heavy chain has been substituted, wherein said first and said second heavy chains are not substituted in the same positions and wherein the amino acid positions are numbered according the EU-index.

In another embodiment, (i) the first CH3 region has an F405L substitution and the second CH3 region has a K409R substitution, or (ii) the first CH3 region has a K409R substitution and the second CH3 region has an F405L substitution.

According to another embodiment, the multispecific antigen-binding molecule is conjugated to a cytotoxic moiety, a radioisotope, a drug, a cytokine or an RNA silencing vehicle. Preferably, the multispecific antigen-binding molecule is conjugated to a cytotoxic moiety, a radioisotope, or a drug. Preferably, the multispecific antigen-binding molecule is conjugated to a cytotoxic moiety.

The cytotoxic moiety may be selected from the group consisting of duostatin-3, duostatin-5, pyrrolobenzodiazepine or an analog or derivative thereof, IGN-based toxins or an analog or derivative thereof, alpha-amanitin or an analog or derivative thereof, dolastatin or an analog or derivative thereof, taxol ; cytochalasin B; gramicidin D; ethidium bromide; emetine; mitomycin; etoposide; tenoposide; vincristine; vinblastine; colchicin; doxorubicin; daunorubicin; dihydroxy anthracin dione; a tubulin-inhibitor such as maytansine or an analog or derivative thereof; mitoxantrone; mithramycin; actinomycin D; 1-dehydrotestosterone; a glucocorticoid; procaine; tetracaine; lidocaine; propranolol ; puromycin; calicheamicin or an analog or derivative thereof an antimetabolite such as methotrexate, 6 mercaptopurine, 6 thioguanine, cytarabine, fludarabin, 5 fluorouracil, decarbazine, hydroxyurea, asparaginase, gemcitabine, or cladribine; an alkylating agent such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin, carboplatin, duocarmycin A, duocarmycin SA, rachelmycin (CC-1065), or an analog or derivative thereof; an antibiotic such as dactinomycin, bleomycin, daunorubicin, doxorubicin, idarubicin, mithramycin, mitomycin, mitoxantrone, plicamycin, anthramycin (AMC)) ; an antimitotic agent such as an auristatin or an analog or derivative thereof, monomethyl auristatin E or F or an analog or derivative thereof; diphtheria toxin and related molecules such as diphtheria A chain and active fragments thereof and hybrid molecules, ricin toxin such as ricin A or a deglycosylated ricin A chain toxin, cholera toxin, a Shiga-like toxin such as SLT I, SLT II, SLT IIV, LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins such as PAPI, PAPII, and PAP S, momordica charantia inhibitor, curcin, crotin, sapaonaria officinalis inhibitor, gelonin, mitogellin, restrictocin, phenomycin, and enomycin toxins; ribonuclease (RNase); DNase I, Staphylococcal enterotoxin A; pokeweed antiviral protein; diphtherin toxin, Pseudomonas endotoxin and RNAi (i.e., siRNA, shRNA conjugated to an antibody or delivered in a nanoparticle).

According to another embodiment, the cytotoxic moiety is selected from the group consisting of maytansine, calicheamicin, duocarmycin, duostatin, duostatin-3, duostatin-5, rachelmycin (CC-1065), auristatin, monomethyl auristatin E, monomethyl auristatin F, doxorubicin, dolastatin, pyrrolobenzodiazepine, IGN-based toxins, alpha-amanitin, or an analog, derivative, or prodrug of any thereof.

In one embodiment, the cytotoxic moiety, drug or radioisotope is linked to said antibody, or fragment thereof, with a cleavable linker, such as N-succinimydyl 4-(2-pyridyldithio)-pentanoate (SSP), maleimidocaproyl-valine-citrulline-p-aminobenzyloxycarbonyl (mc-vc-PAB) or AV-1 K-lock valine-citrulline.

The term "cleavable linker" as used herein, refers to a subset of linkers that are catalyzed by specific proteases in the targeted cell or in the tumor microenvironment, resulting in release of the cytotoxic agent. Examples of cleavable linkers are linkers based on chemical motifs including disulfides, hydrazones or peptides. Another subset of cleavable linker, adds an extra linker motif between the cytotoxic agent and the primary linker, i.e. the site that attaches the linker-drug combination to the antibody. In some embodiments, the extra linker motif is cleavable by a cleavable agent that is present in the intracellular environment (e. g. within a lysosome or endosome or caveola). The linker can be, e. g. a peptidyl linker that is cleaved by an intracellular peptidase or protease enzyme, including but not limited to, a lysosomal or endosomal protease. In some embodiments, the peptidyl linker is at least two amino acids long or at least three amino acids long. Cleaving agents can include cathepsins B and D and plasmin, all of which are known to hydrolyze dipeptide drug derivatives resulting in the release of active drug inside the target cells (see e. g. Dubowchik and Walker, 1999, Pharm. Therapeutics 83:67-123). In a specific embodiment, the peptidyl linker cleavable by an intracellular protease is a Val-Cit (valine-citrulline) linker or a Phe-Lys (phenylalanine-lysine) linker (see e.g. US6214345, which describes the synthesis of doxorubicin with the Val-Cit linker). An advantage of using intracellular proteolytic release of the therapeutic agent is that the agent is typically attenuated when conjugated and the serum stabilities of the conjugates are typically high.

In another embodiment, the cytotoxic agent, drug or radioisotope is linked to said antibody, or fragment thereof, with a non-cleavable linker, such as succinimidyl-4(N-maleimidomethyl)cyclohexane-1-carboxylate (MCC) or maleimidocaproyl (MC).

The term "noncleavable linker" as used herein, refers to a subset of linkers which, in contrast to cleavable linkers, do not comprise motifs that are specifically and predictably recognized by intracellular or extracellular proteases. Thus, ADCs based on non-cleavable linkers are not released or cleaved form the antibody until the complete antibody-linker-drug complex is degraded in the lysosomal compartment. Examples of a non-cleavable linker are thioethers. In yet another embodiment, the linker unit is not cleavable and the drug is released by antibody degradation.

In a particularly preferred embodiment, the binding of T and E by the multispecific antigen-binding molecule induces internalization of the multispecific antigen-binding molecule to a greater extent than the binding to target T alone.

In another aspect, the present invention relates to multispecific antibodies generated by using technologies or formats such as but not limited to: DuoBody, CrossMab, Triomab, kih IgG common LC, DVD-Ig, 2 in 1-IgG, IgG-scFv, bi-Nanobody, BiTE, TandAbs, DART, DART-Fc, scFv-HSA-scFv, orthoFab-IgG, tetravalent Tv-IgGs, dock-and-lock (DNL) formats such as DNL-Fab3, or fragments such as tandem scFv, tandem scFv-Fc, knob-into-hole IgGs, scFv-Fc knobs-into-holes, scFv-Fc-scFv, F(ab')2, Fab-scFv, (Fab'scFv)2, Diabody, scDiabody, scDiabody-Fc, scDiabody-CH3, or Azymetric scaffold.

In another aspect, the present invention relates to a bispecific antibody fragment of the multispecific antigen-binding molecule, wherein the antibody fragment is a tandem scFv, tandem scFv-Fc, scFv-Fc knobs-into-holes, scFv-Fc-scFv, F(ab')₂, Fab-scFv, (Fab'scFv)₂, Diabody, scDiabody, scDiabody-Fc, scDiabody-C_{H}3.

In another aspect, the present invention relates to the multispecific antigen-binding molecule or the bispecific antibody fragment for use in a method for treating and/or preventing a cancer. The subject treated in such method is preferably a human individual in need of such treatment, such as a cancer patient. In one embodiment, the cancer is breast cancer, including primary, metastatic, and refractory breast cancer.

In some embodiments, the cancer is endometrial/cervical cancer, lung cancer, malignant melanoma, ovarian cancer, pancreatic cancer, prostate cancer, testis cancer, a soft-tissue tumor such as synovial sarcoma, breast cancer, brain tumor, leukemia, lymphoma, mastocytoma, renal cancer, uterine cervix cancer, bladder cancer, esophageal cancer, gastric cancer, or colorectal cancer.

The effective dosages and the dosage regimens for the multispecific antigen-binding molecule depend on the cancer to be treated. An exemplary, non-limiting range for a therapeutically effective amount of a bispecific antibody of the present invention is about 0.1-100 mg/kg, such as about 0.1-50 mg/kg, for example about 0.1-20 mg/kg, such as about 0.1-10 mg/kg, for instance about 0.5, about such as 0.3, about 1, about 3, about 5, or about 8 mg/kg.

In some embodiments, the multispecific antigen-binding molecule may be administered prophylactically in order to reduce the risk of developing cancer, delay the onset of the occurrence of an event in cancer progression, or in an adjuvant setting, and/or to reduce the risk of recurrence when a cancer is in remission.

In one embodiment, the method for treating or preventing a cancer comprises administration of a therapeutically effective amount of the multispecific antigen-binding molecule of the present invention and at least one additional therapeutic agent to a subject in need thereof. In some embodiments, such an additional therapeutic agent may be selected from an antimetabolite, such as methotrexate, 6-mercaptopurine, 6-thioguanine, cytarabine, fludarabine, 5-fluorouracil, decarbazine, hydroxyurea, asparaginase, gemcitabine or cladribine. In other embodiments, such an additional therapeutic agent may be selected from an alkylating agent, such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin. In other embodiments, such an additional therapeutic agent may be selected from an anti-mitotic agent, such as taxanes, for instance docetaxel, and paclitaxel, and vinca alkaloids, for instance vindesine, vincristine, vinblastine, and vinorelbine. In other embodiments, such an additional therapeutic agent may be selected from a topoisomerase inhibitor, such as topotecan or irinotecan, or a cytostatic drug, such as etoposide and teniposide. In other embodiments, such an additional therapeutic agent may be selected from a growth factor inhibitor, such as an inhibitor of ErbB1 (EGFR) (such as an EGFR antibody, e.g. zalutumumab, cetuximab, panitumumab or nimotuzumab or other EGFR inhibitors, such as gefitinib or erlotinib), another inhibitor of ErbB2 (HER2/neu) (such as a HER2 antibody, e.g. trastuzumab, trastuzumab-DMI or pertuzumab) or an inhibitor of both EGFR and HER2, such as lapatinib). In other embodiments, such an additional therapeutic agent may be selected from a tyrosine kinase inhibitor, such as imatinib or lapatinib.

In another aspect, the present invention relates to the multispecific antigen-binding molecule for use in a method of targeting a tumor in a subject, the method comprising administering to the subject the multispecific antigen-binding molecule. Tumors which may be targeted in accordance with the present invention include malignant and non-malignant tumors. Malignant (including primary and metastatic) tumors which may be treated include, but are not limited to, those occurring in the adrenal glands; bladder; bone; breast; cervix; endocrine glands (including thyroid glands, the pituitary gland, and the pancreas); colon; rectum; heart; hematopoietic tissue; kidney; liver; lung; muscle; nervous system; brain; eye; oral cavity; pharynx; larynx; ovaries; penis; prostate; skin (including melanoma); testicles; thymus; and uterus. Examples of such tumors include apudoma, choristoma, branchioma, malignant carcinoid syndrome, carcinoid heart disease, carcinoma (e.g., Walker, basal cell, basosquamous, Brown-Pearce, ductal, Ehrlich tumor, in situ, Krebs 2, Merkel cell, mucinous, non-small cell lung, oat cell, papillary, scirrhous, bronchiolar, bronchogenic, squamous cell, and transitional cell), plasmacytoma, melanoma, chondroblastoma, chondroma, chondrosarcoma, fibroma, fibrosarcoma, giant cell tumors, histiocytoma, lipoma, liposarcoma, mesothelioma, myxoma, myxosarcoma, osteoma, osteosarcoma, Ewing's sarcoma, synovioma, adenofibroma, adenolymphoma, carcinosarcoma, chordoma, mesenchymoma, mesonephroma, myosarcoma, ameloblastoma, cementoma, odontoma, teratoma, thymoma, trophoblastic tumor, adenocarcinoma, adenoma, cholangioma, cholesteatoma, cylindroma, cystadenocarcinoma, cystadenoma, granulosa cell tumor, gynandroblastoma, hepatoma, hidradenoma, islet cell tumor, Leydig cell tumor, papilloma, Sertoli cell tumor, theca cell tumor, leiomyoma, leiomyosarcoma, myoblastoma, myoma, myosarcoma, rhabdomyoma, rhabdomyosarcoma, ependymoma, ganglioncuroma, glioma, mcdulloblastoma, meningioma, neurilemnnoma, neuroblastoma, neuroepithelioma, neurofibroma, neuroma, paraganglioma, paraganglioma nonchromaffin, angiokeratoma, angiolymphoid hyperplasia with eosinophilia, angioma sclerosing, angiomatosis, glomangioma, hemangioendothelioma, hemangioma, hemangiopericytoma, hemangiosarcoma, lymphangioma, lymphangiomyorna, lymphangiosarcoma, pinealoma, carcinosarcoma, chondrosarcoma, cystosarcoma phyllodes, fibrosarcoma, hemangiosarcoma, leiomyosarcoma, leukosarcoma, liposarcoma, lymphangiosarcoma, myosarcoma, myxosarcoma, ovarian carcinoma, rhabdomyosarcoma, sarcoma (e.g., Ewing's experimental, Kaposi's, and mast-cell), neoplasms and for other such cells. The administration may include intradermal, intramuscular, intraperitoneal, intravenous, subcutaneous, intranasal, epidural, and oral routes.

Similarly, the invention relates to a method for killing a tumor cell expressing a tumor-associated target molecule, such as HER2, comprising administration, to an individual in need thereof, of an effective amount of the multispecific antigen-binding molecule, such as a bispecific antibody, of the present invention, such as an antibody drug-conjugate (ADC).

In one embodiment, said tumor cell is involved in a form of cancer selected from the group consisting of: breast cancer, prostate cancer, non-small cell lung cancer, bladder cancer, ovarian cancer, gastric cancer, colorectal cancer, esophageal cancer and squamous cell carcinoma of the head & neck, cervical cancer, pancreatic cancer, testis cancer, malignant melanoma, and a soft-tissue cancer (e.g. synovial sarcoma).

In yet another aspect, the present invention relates to a pharmaceutical composition comprising the multispecific antigen-binding molecule as an active ingredient. Advantageously, such pharmaceutical composition is formulated with suitable excipients, such as antioxidants, anti-bacterial agents, chelating agents, buffering agents, coloring agents, flavoring agents, diluting agents, emulsifying agents and/or suspending agents. The pharmaceutical composition may be administered by infusion, by bolus injection, by absorption through epithelial or mucocutaneous linings. In some embodiments, the pharmaceutical composition of the present invention may comprise one or more additional pharmaceutically active ingredients, such cytotoxic substances or anti-cancer drugs.

In another aspect, the present invention relates to a method of treatment of a disease comprising administering the multispecific antigen-binding molecule of the present invention or the pharmaceutical composition of the present invention to a subject in need thereof.

In another aspect, the present invention relates to nucleic acids, such as DNA molecules, encoding a multispecific antigen-binding molecule according to the present invention. The nucleic acid may encode heavy and light chains of bispecific antigen-binding molecule, such as an antibody, of the present invention.

In another aspect, the present invention relates to an expression vector, or a set of expression vectors, containing said nucleic acid and being capable of expressing said nucleic acid in prokaryotic or eukaryotic host cell lines. The heavy and light chain of the antibody may be encoded by the same vector or by different vectors depending on the bispecific antibody technology used. Such expression vectors may be used for recombinant production of antibodies of the invention.

An expression vector in the context of the present invention may be any suitable vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In one embodiment, the antibody-encoding nucleic acids are comprised in a naked DNA or RNA vector, including, for example, a linear expression element, a compacted nucleic acid vector, a plasmid vector such as pBR322, pUC 19/18, or pUC 118/119, a "midge" minimally-sized nucleic acid vector, or as a precipitated nucleic acid vector construct, such as a CaP04-precipitated construct.

In another aspect, the present invention relates to prokaryotic or eukaryotic host cell lines comprising said vectors. A host cell is a cell into which the expression vector has been introduced, i.e. the expression vector encoding a homodimeric monospecific precursor molecules when Duobody technology is used to generate the bispecific antigen-binding molecule, of the present invention. or the single host cells comprising nucleic acids encoding the bispecific molecules of the invention. Recombinant host cells include, for example, transfectomas, such as CHO cells, HEK293 cells, NS/0 cells, and lymphocytic cells.

In another embodiment the present invention relates to anti-idiotypic antibodies raised against the multispecific antigen-binding molecule of the invention as defined above. An anti-idiotypic (Id) antibody is an antibody which recognizes unique determinants generally associated with the antigen-binding site of an antibody. An Id antibody may be prepared by immunizing an animal with the multispecific antigen-binding molecule, such as a bispecific antibody as describe above to which an anti-Id is being prepared. The immunized animal typically can recognize and respond to the idiotypic determinants of the immunizing bispecific antibody by producing an antibody to these idiotypic determinants (the anti-Id antibody).

In one embodiment the anti-idiotypic antibody is used for detecting the level of a multispecific antigen-binding molecule as defined above, in a sample.

Embodiment 1. A multispecific antigen-binding molecule comprising a first antigen-binding domain and a second antigen-binding domain, wherein the first antigen-binding domain specifically binds a target molecule (T), and wherein the second antigen-binding domain specifically binds an internalizing effector protein (E), and wherein the second antigen-binding domain has a dissociation constant K_{D} value with E of between 10⁻⁹ and 10⁻⁸ M.

Embodiment 2. The multispecific antigen-binding molecule according to embodiment 1, comprising i) a first binding arm which comprises the first antigen-binding domain and ii) a second binding arm which comprises the second antigen-binding domain.

Embodiment 3. The multispecific antigen-binding molecule according to embodiments 1 or 2, wherein the multispecific antigen-binding molecule is a bispecific antigen-binding molecule.

Embodiment 4. The multispecific antigen-binding molecule according to any of the preceding embodiments, wherein the second antigen-binding domain has a dissociation constant K_{D} value with E higher than 10⁻⁹ and lower than 10⁻⁸ M.

Embodiment 5. The multispecific antigen-binding molecule according to any of the preceding embodiments, wherein the second antigen-binding domain has a dissociation constant K_{D} value with E of between 2.0x10⁻⁹ and 9.0x10⁻⁹ M.

Embodiment 6. The multispecific antigen-binding molecule according to any of the preceding embodiments, wherein the second antigen-binding domain has a dissociation constant K_{D} value with E of between 2.0x10⁻⁹ and 7.3x10⁻⁹ M.

Embodiment 7. The multispecific antigen-binding molecule according to any of the preceding embodiments, wherein E is a cell surface-expressed molecule that is internalized into the cell.

Embodiment 8. The multispecific antigen-binding molecule according to embodiment 7, wherein the multispecific antigen-binding molecule is internalized into the cell by way of binding to E only in the presence of the target molecule (T).

Embodiment 9. The multispecific antigen-binding molecule according to embodiments 7 or 8, wherein the multispecific antigen-binding molecule is internalized into the cell by way of binding to E only when the first domain is specifically bound to the target molecule (T).

Embodiment 10. The multispecific antigen-binding molecule according to any of the preceding embodiments, which upon binding to E internalizes more efficiently into cells expressing T as compared to cells not expressing T.

Embodiment 11. The multispecific antigen-binding molecule according to any of the preceding embodiments, which upon binding to T internalizes more efficiently into cells expressing E as compared to cells not expressing E.

Embodiment 12. The multispecific antigen-binding molecule according to any of the preceding embodiments, which upon binding to E is transported to the lysosomal compartment in cells expressing T.

Embodiment 13. The multispecific antigen-binding molecule according to any of the preceding embodiments, which upon binding to E is more efficiently transported to the lysosomal compartment in cells expressing T as compared to cells not expressing T.

Embodiment 14. The multispecific antigen-binding molecule according to any of the preceding embodiments, which upon binding to T is more efficiently transported to the lysosomal compartment in cells expressing E as compared to cells not expressing E.

Embodiment 15. A multispecific antigen-binding molecule according to any of the preceding embodiments, wherein E is selected from the group consisting of CD63, MHC-I, Kremen-1, Kremen-2, LRP5, LRP6, transferrin receptor, LDLr, MAL, V-ATPase and ASGR.

Embodiment 16. The multispecific antigen-binding molecule according to any of the preceding embodiments, wherein E is CD63.

Embodiment 17. The multispecific antigen-binding molecule according to any of embodiments 1-6, wherein E is a soluble ligand that is internalized into a cell via the interaction between E and an internalizing cell surface-expressed receptor molecule.

Embodiment 18. The multispecific antigen-binding molecule according to any of the preceding embodiments, wherein T is a cell surface-expressed target molecule.

Embodiment 19. The multispecific antigen-binding molecule according to any of the preceding embodiments, wherein T is a tumor-associated antigen.

Embodiment 20. The multispecific antigen-binding molecule according to any of the preceding embodiments, wherein T is HER2.

Embodiment 21. The multispecific antigen-binding molecule according to any of the preceding embodiments, wherein the first and/or the second antigen-binding domain comprises at least one antibody variable region.

Embodiment 22. The multispecific antigen-binding molecule according to any of the preceding embodiments, wherein the multispecific antigen-binding molecule is a multispecific antibody, preferably a bispecific antibody, or a multispecific, preferably bispecific, antibody fragment or recombinantly engineered part thereof.

Embodiment 23. The multispecific antigen-binding molecule according to any of the preceding embodiments, wherein the multispecific antigen-binding molecule is a bispecific antibody comprising a first binding arm comprising the first antigen-binding domain and a second binding arm comprising said second antigen-binding domain.

Embodiment 24. The multispecific antigen-binding molecule according to embodiment 23, wherein said first antigen-binding domain comprises a first heavy chain variable sequence (VH) and a first light chain variable sequence (VL), and said second antigen-binding domain comprises a second heavy chain variable sequence (VH) and a second light chain variable sequence (VL) and wherein said variable sequences each comprise three CDR sequences, CDR1, CDR2 and CDR3.

Embodiment 25. The multispecific antigen-binding molecule according to embodiments 23 or 24 wherein (i) said first binding arm comprises a first heavy chain comprising a first heavy chain variable sequence (VH) and a first heavy chain constant sequence (CH), and a first light chain comprising a first light chain variable sequence (VL) and a first light chain constant sequence (CL), and (ii) said second binding arm comprises a second heavy chain comprising a second heavy chain variable sequence (VH) and a second heavy chain constant sequence (CH), and a second light chain comprising a second light chain variable sequence (VL) and a second light chain constant sequence (CL).

Embodiment 26. The multispecific antigen-binding molecule according to any of embodiments 23-25, wherein the first binding arm is derived from a chimeric antibody or from a humanized antibody or from a human antibody.

Embodiment 27. The multispecific antigen-binding molecule according to any of embodiments 23-26, wherein the second binding arm is derived from a chimeric antibody or from a humanized antibody or from a human antibody.

Embodiment 28. The multispecific antigen-binding molecule according to any of the preceding embodiments, which is a bispecific antibody, wherein the bispecific antibody is a full-length antibody, preferably an IgG1 antibody.

Embodiment 29. The multispecific antigen-binding molecule according to any of the preceding embodiments, wherein said second antigen-binding domain has one or more mutations that modulates the affinity of the second antigen-binding domain with E.

Embodiment 30. The multispecific antigen-binding molecule according to embodiment 29, wherein said second antigen-binding domain is derived from an antibody having one or more mutations in the VH and/or VL that modulates the affinity of the second antigen-binding domain with E.

Embodiment 31. The multispecific antigen-binding molecule according to embodiment 30, wherein the mutation is a single amino acid substitution, preferably a single amino acid histidine substitution.

Embodiment 32. The multispecific antigen-binding molecule according to any of the preceding embodiments, wherein said second domain comprises:
a. VH CDRs 1, 2, and 3 as provided in SEQ ID Nos: 2, 3, and 4, respectively, and VL CDRs 1, 2, and 3 as provided in SEQ ID Nos: 9, 7, and 8, respectively, or
b. VH CDRs 1, 2, and 3 as provided in SEQ ID Nos: 2, 10, and 4, respectively, and VL CDRs 1, 2, and 3 as provided in SEQ ID Nos: 6, 7, and 8, respectively, or
c. VH CDRs 1, 2, and 3 as provided in SEQ ID Nos: 2, 11, and 4, respectively, and VL CDRs 1, 2, and 3 as provided in SEQ ID Nos: 6, 7, and 8, respectively, or
d. VH CDRs 1, 2, and 3 as provided in SEQ ID Nos: 2, 12, and 4, respectively, and VL CDRs 1, 2, and 3 as provided in SEQ ID Nos: 6, 7, and 8, respectively.

Embodiment 33. The multispecific antigen-binding molecule according to embodiment 32, wherein said second binding domain comprises VH CDRs 1, 2, and 3 as provided in SEQ ID Nos: 2, 12, and 4, respectively, and VL CDRs 1, 2, and 3 as provided in SEQ ID Nos: 6, 7, and 8, respectively.

Embodiment 34. The multispecific antigen-binding molecule according to any of the preceding embodiments, wherein said first and second antigen-binding domains are each a pair of an antibody heavy chain variable domain and an antibody light chain variable domain.

Embodiment 35. The multispecific antigen-binding molecule according to any of the preceding embodiments, wherein the antigen-binding molecule is a tumor-associated target (T)xCD63 bispecific antibody.

Embodiment 36. The multispecific antigen-binding molecule according to any of the preceding embodiments, wherein the antigen-binding molecule is a HER2xCD63 bispecific antibody.

Embodiment 37. The multispecific antigen-binding molecule according to any of the preceding embodiments, wherein the antigen-binding molecule has an EC₅₀ value for binding to tumor-associated target (T)-expressing cells, such as HER2-expressing cells, of lower than 5.0 µg/ml, such as lower than 0.5 µg/ml, as determined by flow cytometry.

Embodiment 38. The multispecific antigen-binding molecule according to any of the preceding embodiments, wherein K_{D} is determined by biolayer interferometry at 30 °C.

Embodiment 39. The multispecific antigen-binding molecule according to any of the preceding embodiments, wherein the antigen-binding molecule is a bispecific antibody comprising:
i) a first binding arm comprising a first heavy chain comprising a first heavy chain constant sequence (CH), said first CH comprising a first CH3 region, and
ii) a second binding arm comprising a second heavy chain comprising a second heavy chain constant sequence (CH), said second CH comprising a second CH3 region,
wherein the sequences of said first and second CH3 regions are different and are such that a heterodimeric interaction between said first and second binding arm is stronger than a homodimeric interaction of each of said first and second binding arms.

Embodiment 40. The multispecific antigen-binding molecule according to embodiment 39, wherein in said first heavy chain CH3 region at least one of the amino acids in a position corresponding to positions T366, L368, K370, D399, F405, Y407 or K409 of human IgG1 heavy chain has been substituted, and in said second heavy chain CH3 region at least one of the amino acids in a position corresponding to positions T366, L368, K370, D399, F405, Y407 or K409 of human IgG1 heavy chain has been substituted, and wherein said first and said second heavy chains are not substituted in the same positions and wherein the amino acid positions are numbered according the EU-index.

Embodiment 41. The multispecific antigen-binding molecule according to embodiment 40, wherein (i) the first CH3 region has an F405L substitution and the second CH3 region has a K409R substitution, or (ii) the first CH3 region has a K409R substitution and the second CH3 region has an F405L substitution.

Embodiment 42. The multispecific antigen-binding molecule according to any of the preceding embodiments, wherein the multispecific antigen-binding molecule is conjugated to a cytotoxic moiety, a radioisotope or a drug.

Embodiment 43. The multispecific antigen-binding molecule according to embodiment 42, wherein the cytotoxic moiety is selected from the group consisting of maytansine, calicheamicin, duocarmycin, duostatin, duostatin-3, duostatin-5, rachelmycin (CC-1065), auristatin, monomethyl auristatin E, monomethyl auristatin F, doxorubicin, dolastatin, pyrrolobenzodiazepine, IGN-based toxins, alpha-amanitin, or an analog, derivative, or prodrug of any thereof.

Embodiment 44. The multispecific antigen-binding molecule according to any of the preceding embodiments, wherein binding of T and E by the multispecific antigen-binding molecule induces internalization of the multispecific antigen-binding molecule to a greater extent than the binding of T alone.

Embodiment 45. A bispecific antibody fragment of the multispecific antigen-binding molecule according to any of the preceding embodiments, wherein the antibody fragment is a tandem scFv, tandem scFv-Fc, scFv-Fc knobs-into-holes, scFv-Fc-scFv, F(ab')₂, Fab-scFv, (Fab'scFv)₂, Diabody, scDiabody, scDiabody-Fc, scDiabody-C_{H}3, or azymetric scaffold.

Embodiment 46. A multispecific antigen-binding molecule according to any of embodiments 1-44 or a bispecific antibody fragment according to embodiment 45 for use in a method for treating and/or preventing a cancer.

Embodiment 47. The multispecific antigen-binding molecule for use according to embodiment 46, wherein the cancer is endometrial/cervical cancer, lung cancer, malignant melanoma, ovarian cancer, pancreatic cancer, prostate cancer, testis cancer, a soft-tissue tumor such as synovial sarcoma, breast cancer, brain tumor, leukemia, lymphoma, mastocytoma, renal cancer, uterine cervix cancer, bladder cancer, esophageal cancer, gastric cancer, or colorectal cancer.

Embodiment 48. A multispecific antigen-binding molecule according to any of embodiments 1-44 for use in a method of targeting a tumor in a subject, the method comprising administering to the subject the multispecific antigen-binding molecule.

Embodiment 49. A pharmaceutical composition comprising the multispecific antigen-binding molecule according to any of embodiments 1-44 as an active ingredient.

Embodiment 50. A method of treatment of a disease comprising administering the multispecific antigen-binding molecule according to any one of the preceding embodiments 1-44 or the pharmaceutical composition according to embodiment 49 to a subject in need thereof.

Embodiment 51. A nucleic acid encoding a multispecific antigen-binding molecule according to any of embodiments 1-44.

Embodiment 52. An expression vector containing the nucleic acid according to embodiment 51 capable of expressing said nucleic acid in prokaryotic or eukaryotic host cell lines.

Embodiment 53. A prokaryotic or eukaryotic host cell line comprising a vector according to embodiment 52.

### EXAMPLES

### Example 1: Antibody generation, site-directed mutagenesis and Duostatin-3 conjugation

Cloning and production of the human HER2 antibody IgG1-153 has been described elsewhere; de Goeij B.E.C.G. MAbs, 2014. 6(2): p. 392-402. The variable domain heavy and light chain regions of the mouse monoclonal CD63 antibody 2192 (see Table 1 below, SEQ ID Nos. 1 and 5) were obtained from hybridoma 2.19 (Metzelaar M.J. Virchows Arch B Cell Pathol Incl Mol Pathol, 1991. 61(4): p. 269-77), by 5'-RACE of the variable regions from hybridoma-derived RNA and sequencing. Variable regions were cloned in the mammalian expression vector pcDNA3.3 (Invitrogen) containing the relevant human light chain constant domains (codon optimized, Invitrogen) with the relevant human heavy chain constant domain mutations (K409R or F405L). The human-mouse chimeric CD63 antibody was referred to as wild type IgG1-CD63. IgG1-CD63 antibody mutations were introduced in the variable domains either by site directed mutagenesis or direct gene synthesis, with the aim to generate a panel of IgG1-CD63 affinity variants. The amino acid mutations were indicated in the antibody names (i.e. anti-CD63-N74H has an asparagine to histidine mutation at amino acid position 74 of the heavy chain (SEQ ID No. 1, Table 1), anti-CD63-LN54H has a asparagine to histidine mutation at position 54 of the light chain, as numbered in SEQ ID No. 5, Table 1. Antibodies were produced by co-transfection of heavy chain and light chain vectors and transient expression in HEK-293 freestyle cells (Invitrogen) as described by Vink T. Methods, 2014. 65(1): p. 5-10. Bispecific antibodies (Duobody) were made by controlled Fab-arm exchange as described by Labrijn A.F. Nat Protoc, 2014. 9(10): p. 2450-63. The HIV gp120-specific human antibody IgG1-b12 was included as isotype control, see; Parren P.W.H.I. AIDS, 1995. 9(6): p. F1-6.

Duostatin-3 conjugated antibodies were generated by covalent conjugation of valine-citrulline-duostatin-3 (Duo3) on antibody lysine groups of IgG1-HER2-F405L and IgG1-b12-F405L as described by de Goeij B.E.C.G. Mol Cancer Ther. 2015. 14(5):1130-40. The bispecific ADCs bsHER2xCD63-Duo3 and bsHER2xb12-Duo3 were generated by Fab-arm exchange of the Duo3-conjugated antibody IgG1-HER2-F405L-Duo3 with unconjugated IgG1-CD63-K409R or IgG1-b12-K409R. The bispecific ADC bsCD63xb12-Duo3 was generated by Fab-arm exchange of IgG1-b12-F405L-Duo3 with IgG1-CD63-K409R. All bispecific ADCs had a DAR of 1. To generate control ADCs with a DAR of 1, IgG1-HER2-F405L-Duo3 and IgG1-b12-F405L-Duo3 were Fab-arm exchanged with IgG1-HER2-K409R and IgG1-b12-K409R, to generate IgG1-HER2-Duo3 and IgG1 b12-Duo3, respectively. The DAR of the ADCs was determined by hydrophobic interaction chromatography (HIC).

**Table 1 - Heavy chain variable region (VH), light chain variable region (VL) and CDR sequences of the anti-CD63 antibody 2192**

| | | |
|---|---|---|
| SEQ ID No: 1 | VH 2192 Amino acid positions 20-143 | 20        30        40        50 ;         ;         ;         ; EVQLQQSGPE LVKPGASVKM SCKASGYTFT SYVMHWVRQT 60        70        80        90 ;         ;         ;         ; PGQGLEWIGY ITPYNDGTKY NEKFKGKATL TSDKSSSTAY 100       110       120       130 ;         ;         ;         ; MELSSLTSED SAVYYCVGGD NYYYAMDYWG QGTSVTVSAA 140 ; STKG |
| SEQ ID No: 2 | VH 2192, CDR1 | GYTFTSYV |
| SEQ ID No: 3 | VH 2192, CDR2 | ITPYNDGT |
| SEQ ID No: 4 | VH 2192, CDR3 | VGGDNYYYAMDY |
| SEQ ID No: 5 | VL 2192 Amino acid positions 21-134 | 21        30         40         50 ;         ;          ;          ; NIMVTQSPS SLAVSAGEKV TMSCKSSQSV LYSSNQKNYL 60         70         80         90 ;          ;          ;          ; AWYQQKPGQS PKLLIYWAST RVSGVPDRFT GSGSGTDFTL 100        110        120        130 ;          ;          ;          ; TISRVQAEDL AVYYCHQYFS SFTFGSGTKL EIKRT |
| SEQ ID No:6 | VL 2192, CDR1 | QSVLYSSNQKNY |
| SEQ ID No: 7 | VL 2192, CDR2 | WAS |
| SEQ ID No: 8 | VL 2192, CDR3 | HQYFSSFT |
| SEQ ID No: 9 | LN54H mutation in VL CDR1 | QSVLYSSHQKNY |
| SEQ ID No: 10 | T71H mutation in VH CDR2 | IHPYNDGT |
| SEQ ID No: 11 | P72H mutation in VH CDR2 | ITHYNDGT |
| SEQ ID No: 12 | N74H mutation in VH CDR2 | ITPYHDGT |

### Example 2: CD63 binding ELISA

To improve efficacy of ADCs, a bispecific ADC was generated that specifically binds to a target protein (T) with one Fab-arm, while its second Fab-arm binds an effector protein (E), that facilitates internalization and lysosomal delivery of the cytotoxic payload. The resulting bsADC should induce cytotoxicity in cells that express both T and E.Some cytotoxicity may also be induced in cells that express T but not E, whereas the bsADC should not induce cytotoxicity in cells that express E but not TCD63 is used as effector protein (E). To ensure tumor specificity of the bsAb, the anti-CD63 arm (E) of the bsAb should preferably not bind and internalize in the absence of the tumor-specific arm (T) or only do this to a very limited degree. A panel of IgG1-CD63 variants with mutations in the variable region was generated as described supra. The IgG1-CD63 antibody variants were screened for binding to soluble CD63 with ELISA. In short, ELISA plates (Greiner) were coated overnight at 4⁰C with 0.8 µg/mL goat anti-human IgG (Jackson). The plates were blocked with 2% chicken serum and incubated with 1 µg/mL histidine-mutated variants of anti-CD63 mAb 2192. Serially diluted (1-0.0005 µg/mL) recombinant human CD63 (Creative Biomart) was added followed by 1 µg/mL mouse anti-poly-histidine-biotin (R&D). The reaction was visualized using ABTS and stopped with oxalic acid. Fluorescence at 405 nm was measured and depicted using GraphPad Prism 6 software.

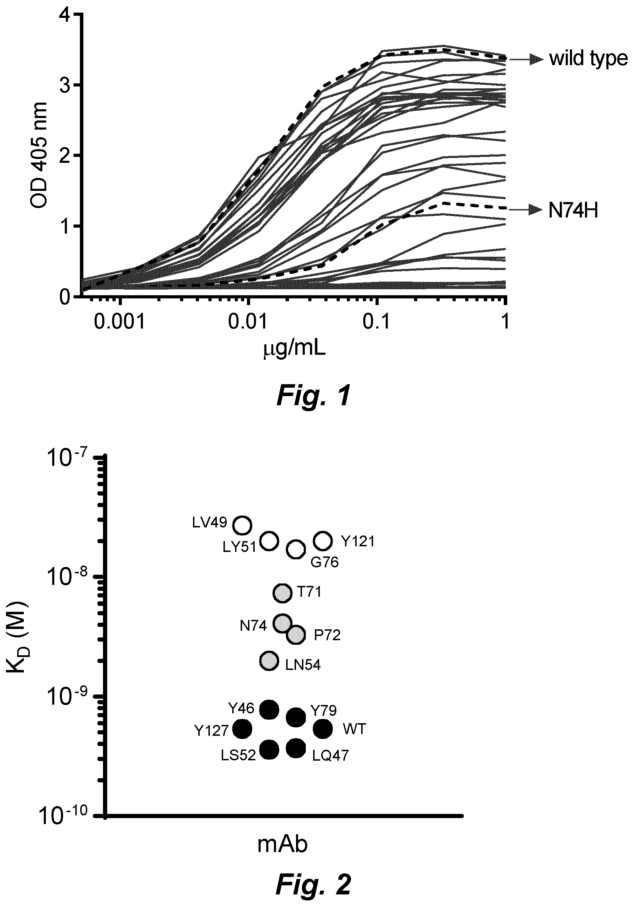

As seen in Figure 1, a wide variety in CD63 binding curves was found for the different histidine mutated anti-CD63 antibodies. For some of the affinity variants the binding to CD63 was comparable to wild type (wt) IgG1-CD63 (referred to as wt IgG1-CD63), while others showed partial or complete loss of binding.

### Example 3: CD63 affinity measurements

The binding kinetics of anti-CD63 antibodies to recombinant human CD63 second extracellular domain (Ala 103-Val 203) fused with a polyhistidine tag at the C-terminus and a signal peptide at the N-terminus (Creative BioMart) was assessed using label-free Bio-Layer Interferometry on an Octet HTX (ForteBio). Wt IgG1-CD63 or affinity variants thereof were immobilized for 1000 s on Anti-Human IgG Fc Capture Biosensors (ForteBio) at 1 µg/mL. Association and dissociation kinetics of human His-tagged CD63 (100 nM, 50 nM, 25 nM and 12.5 nM, concentrations were calculated using the predicted molecular weight of 13 kDa) were determined in Sample Diluent (ForteBio), using an association time of 1000 s, a dissociation time of 2000 s and a shaker speed of 1000 rpm at 30°C. Data traces were corrected using a reference sensor exposed to Sample Diluent only during the association and dissociation steps, the Y-axis was aligned to baseline and Inter-step correction as well as Savitzky-Golay filtering were applied. The association rate constant Kₒₙ (1/Ms), dissociation rate constant K_{dis} (1/s) and equilibrium dissociation constant K_{D} (M) were determined with ForteBio Data Analysis Software v8.1, using the 1:1 model and a global full fit. A dissociation time of 1000 s was used for the calculation of the K_{D}, except for the affinity variants T71, P72, N74, Y121, LV49 and LY51 for which a dissociation time of 200 s was used.

A broad range of Ab-affinities was measured ranging from 3.6 x 10⁻¹⁰ - 2.7 x 10⁻⁸ M for the different anti-CD63 antibody variants (see Figure 2 and Table 2). Thus, by introducing single amino acid histidine substitutions it was possible to reduce the affinity of wt IgG1-CD63.

**Table 2**

| | K_{D} (M) | kₒₙ (1/Ms) | k_{off} (1/s) |
|---|---|---|---|
| WT | 5.4 x 10⁻¹⁰ | 2.0 x 10⁵ | 1.1 x 10⁻⁴ |
| Y46 | 7.8 x 10⁻¹⁰ | 2.0 x 10⁵ | 1.6 x 10⁻⁴ |
| Y79 | 6.7 x 10⁻¹⁰ | 2.1 x 10⁵ | 1.4 x 10⁻⁴ |
| Y127 | 5.4 x 10⁻¹⁰ | 2.0 x 10⁵ | 1.1 x 10⁻⁴ |
| LQ47 | 3.7 x 10⁻¹⁰ | 1.8 x 10⁵ | 6.7 x 10⁻⁵ |
| LS52 | 3.6 x 10⁻¹⁰ | 1.7 x 10⁵ | 6.3 x 10⁻⁵ |
| T71 | 7.3 x 10⁻⁹ | 1.5 x 10⁵ | 1.1 x 10⁻³ |
| P72 | 3.3 x 10⁻⁹ | 1.7 x 10⁵ | 5.7 x 10⁻⁴ |
| N74 | 4.1 x 10⁻⁹ | 1.7 x 10⁵ | 6.9 x 10⁻⁴ |
| LN54 | 2.0 x 10⁻⁹ | 1.9 x 10⁵ | 3.9 x 10⁻⁴ |
| G76 | 1.7 x 10⁻⁸ | 2.2 x 10⁴ | 3.8 x 10⁻⁴ |
| Y121 | 2.0 x 10⁻⁸ | 7.0 x 10⁴ | 1.4 x 10⁻³ |
| LV49 | 2.7 x 10⁻⁸ | 3.7 x 10⁴ | 2.0 x 10⁻³ |
| LY51 | 2.0 x 10⁻⁸ | 9.8 x 10⁴ | 1.9 x 10⁻³ |

### Example 4: Cytotoxicity of affinity variants of bsCD63xHER2-Duo3 ADCs and affinity variants of monovalent bsCD63xb12-Duo3 ADCs using HCC1954, SK-OV-3 and Colo205 cells

Binding of the bsADC to tumor cells expressing both the target protein (T) and the effector protein (E), should preferentially result in cytotoxicity. However, in absence of tumor-associated target protein (T), the bsADC should preferably not induce cytotoxicity. A number of bsADCs were generated targeting CD63 (E) and HER2 (T), using the different anti-CD63 affinity variants. The same anti-CD63 affinity variants were also used to generate bsADCs targeting CD63 and HIV gp120. HIV gp120 is a viral protein that is not expressed on the tested tumor cells. Therefore bsADCs targeting CD63 and gp120 (i.e. bsADCs containing binding domains derived from a CD63 antibody and the gp120-specific antibody IgG1-b12), can only bind to CD63, which reflects the activity of the ADC on normal tissue that lacks expression of T.

Cytotoxicity of the bsADCs was tested using HCC1954, SK-OV-3 and Colo205 cells. Cells were seeded in 96-well tissue culture plates (5,000 cells/well) and incubated for 6 hours at 37°C. Serially diluted ADCs (10-0.0005 µg/mL) were added and the cells were incubated for 4 days at 37°C. Cell viability was assessed using CellTiter-GLO (Promega), according to the manufacturer's guidelines. The percentage of viable cells was depicted as a percentage relative to untreated cells (0% cell death) and staurosporin-treated cells (100% cell death). Percentage viable cells = (RFU ADC treated cells - RFU staurosporin-treated cells) x 100 / (RFU untreated cells - RFU staurosporin-treated cells) RFU = relative fluorescence units

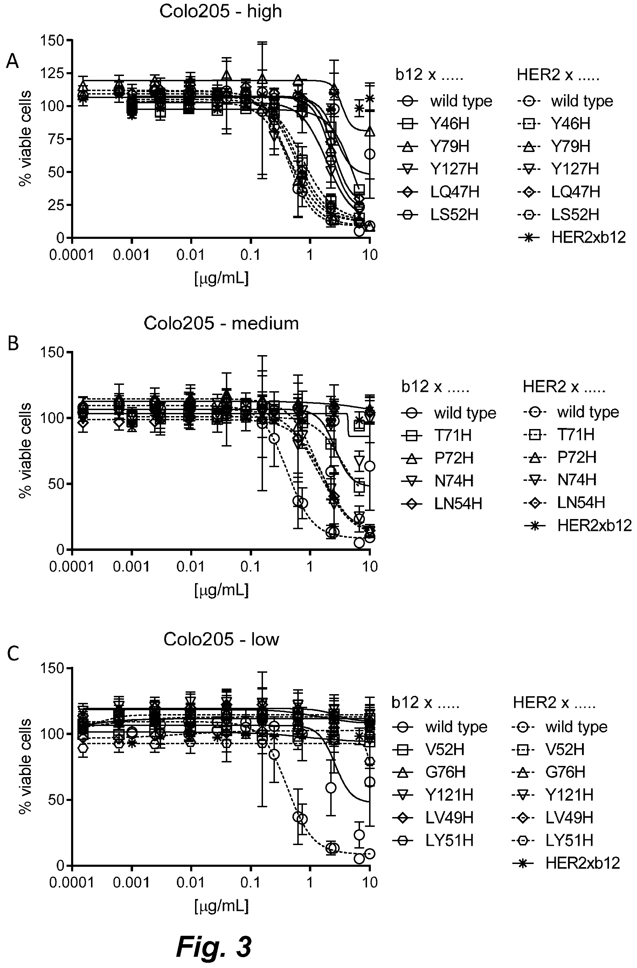

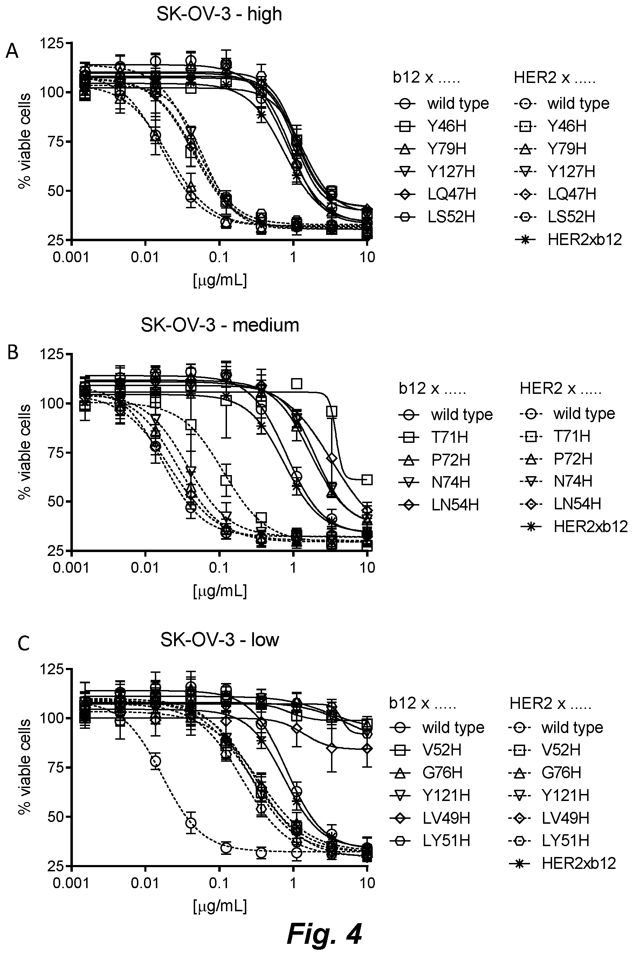

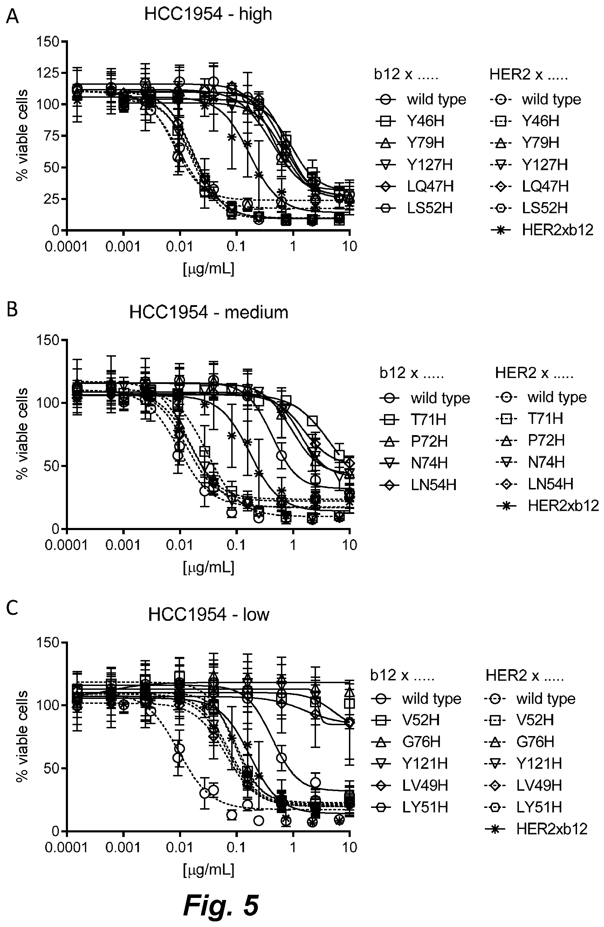

Figures 3-5 show cell viability after 4 days treatment with serially diluted ADCs as percentage compared to untreated cells. Data shown are mean ± standard deviation of at least two different experiments. IC₅₀ values for cytotoxicity were determined using GraphPad Prism 6 software and depicted in Table 3.

**Table 3 - IC50 values**

| | HCC1954 IC50 (µg/mL) **b12x...** | HCC1954 IC50 (µg/mL) **HER2x...** | SKOV3 IC50 (µg/mL) **b12x...** | SKOV3 IC50 (µg/mL) **HER2x...** | Colo205 IC50 (µg/mL) **b12x...** | Colo205 IC50 (µg/mL) **HER2x...** |
|---|---|---|---|---|---|---|
| WT | 0.424 | 0.009 | 0.815 | 0.017 | 2.610 | 0.416 |
| HER2xb12 | - | 0.172 | - | 0.762 | - | 10.000 |
| CD63 variant↓: | | | | | | |
| Y46 | 0.900 | 0.016 | 1.370 | 0.045 | 5.643 | 0.754 |
| Y79 | 0.420 | 0.008 | 0.930 | 0.021 | 3.407 | 0.432 |
| Y127 | 0.664 | 0.017 | 1.079 | 0.061 | 1.889 | 0.472 |
| LQ47 | 0.753 | 0.017 | 1.112 | 0.048 | 2.432 | 0.587 |
| LS52 | 0.662 | 0.018 | 1.129 | 0.047 | 2.248 | 0.567 |
| T71 | 4.886 | 0.030 | 3.803 | 0.115 | 10.000 | 2.228 |
| P72 | 0.920 | 0.014 | 1.719 | 0.028 | 10.000 | 1.402 |
| N74 | 1.350 | 0.017 | 1.988 | 0.037 | 3.927 | 1.346 |
| LN54 | 1.547 | 0.013 | 3.354 | 0.021 | 10.000 | 1.689 |
| V52 | 10.000 | 0.088 | 0.869 | 0.265 | 10.000 | 10.000 |
| G76 | 10.000 | 0.104 | 3.057 | 0.351 | 1.415 | 10.000 |
| Y121 | 10.000 | 0.073 | 5.463 | 0.259 | 10.000 | 10.000 |
| LV49 | 1.646 | 0.065 | 1.398 | 0.314 | 10.000 | 10.000 |
| LY51 | 5.274 | 0.069 | 4.522 | 0.207 | 10.000 | 10.000 |

BsADCs in Figures 3A, 4A and 5A had the highest affinities for CD63 (ranging from 3.6 x 10⁻¹⁰ - 7.8 x 10⁻¹⁰ M; Table 2), induced cytotoxicity with low IC₅₀ value when tested as bsCD63xHER2-ADC, but also showed some cytotoxicity when tested as bsCD63xb12-ADC. BsADCs in Figures 3B, 4B and 5B had moderate affinities (ranging from 2.0 x 10⁻⁹ - 7.3 x 10⁻⁹M), induced cytotoxicity with low IC₅₀ value when tested as bsCD63xHER2-ADC, and showed limited cytotoxicity when tested as bsCD63xb12-ADC. Antibodies in Figures 3C, 4C and 5C had low affinities (ranging from 1.7 x 10⁻⁸ - 2.7 x 10⁻⁸ M), induced cytotoxicity with poor IC₅₀ value when tested as bsCD63xHER2-ADC, and showed hardly any cytotoxicity when combined when tested as bsCD63xb12-ADC.

To conclude, CD63 antibodies depicted in Figures 3B, 4B and 5B, with affinities ranging from 2.0 x 10⁻⁹ - 7.3 x 10⁻⁹ M, showed most favorable characteristics for enhanced delivery of ADCs.

These antibodies were able to induce cytotoxicity with low IC50 value as bsCD63xHER2-ADC, while inducing limited cytotoxicity as bsCD63xb12-ADC.

### Example 5: Lysosomal co-localization of affinity variants of monovalent bsCD63xb12 ADCs

A confocal microscopy experiment was performed to confirm that CD63xb12 bispecific antibodies with reduced affinity for CD63 show less internalization and lysosomal transport. SK-OV-3 cells were cultured on glass coverslips (Thermo Fisher Scientific) at 37°C for 16 hours. Antibody (2 and 10 µg/mL) was added and cells were incubated for 16 hours at 37°C. Cells were fixed, permeabilized and incubated 45 min with goat anti-human IgG1-FITC (Jackson) to stain for human IgG and mouse anti-human CD107a-APC (BD) to stain for lysosomes. Coverslips were mounted (Calbiochem) on microscope slides and imaged with a Leica SPE-II confocal microscope (Leica Microsystems) equipped with LAS-AF software. 12-bit grayscale TIFF images were analyzed for co-localization using MetaMorph^{®} software (Molecular Devices). Co-localization was depicted as arbitrary units [AU] representing the total pixel intensity of antibody overlapping with the lysosomal marker LAMP1. This value was divided by the total pixel intensity of LAMP1, to correct for differences in cell density between different images.

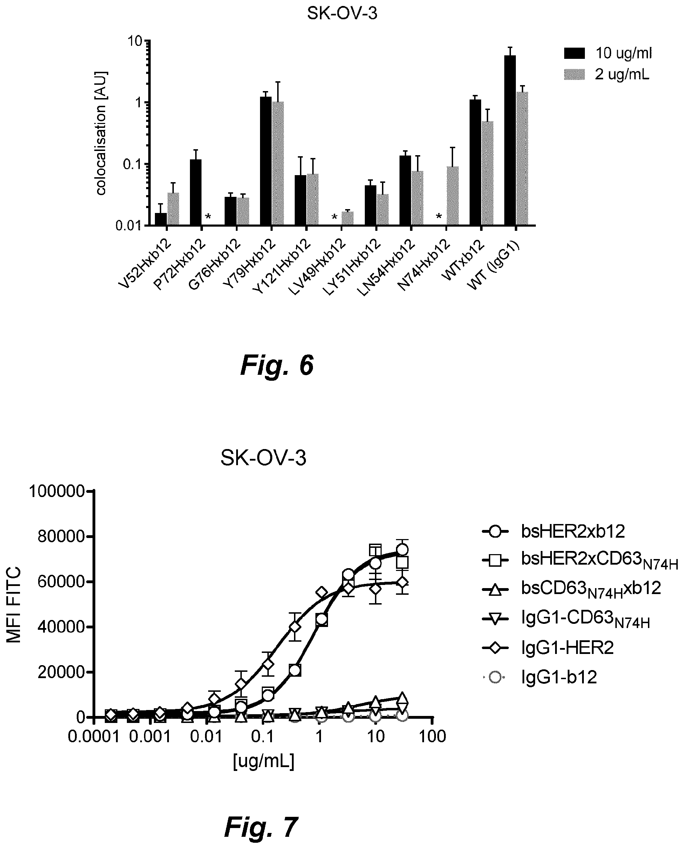

As seen in Figure 6, wild type IgG1-CD63 showed the highest co-localization values, followed by its monovalent counterpart (bsCD63_{WT}xb12) and bsCD63-Y79Hxb12. Lysosomal co-localization values for bsP72Hxb12, bsY121Hxb12, bsLN54Hxb12 and bsN74Hxb12 were ~10 fold lower compared to bsY79Hxb12 and wild type bsCD63xb12. Values for bsV52Hxb12, bsG76Hxb12, bsLV49Hxb12 and bsLY51Hxb12 were even lower, indicating there was hardly any lysosomal transport of the monovalent CD63 antibodies. Samples indicated with an asterisk (*) were not imaged. Data shown are mean ± standard deviation of 3 images.

### Example 6: Binding of bsHER2xCD63_{N74H} to SK-OV-3 cells as determined by flow cytometry

Based on its ability to induce cytotoxicity with low IC50 value as bsCD63xHER2-ADC, while inducing limited cytotoxicity as bsCD63xb12-ADC, clone anti-CD63-N74H was selected for further analysis. The binding of bsHER2xCD63_{N74H} to HER2 positive SK-OV-3 cells was tested using flow cytometry (FACS Canto II, BD Biosciences). Serially diluted antibodies were incubated 30 minutes at 4°C with SK-OV-3 cells. Antibody binding was detected using a Phycoerythrin-conjugated goat-anti-human IgG antibody (Jackson) and samples were analyzed on a flow cytometer. IgG1-b12 was used as isotype control antibody. The resulting data shown in Figure 7 are the mean of two experiments.

As seen in Figure 7, the binding curves of bsHER2xCD63_{N74H} and the monovalent HER2 antibody bsHER2xb12, were identical. This indicates that tumor cell binding of bsHER2xCD63_{N74H} occurs through monovalent binding to HER2. IgG1-CD63_{N74H} and bsCD63_{N74H}xb12 did not show binding to SK-OV-3 cells, which is in line with the low expression of CD63 on the plasma membrane.

### Example 7: mAb-FITC accumulation assay with bsHER2xCD63_{N74H} and bsCD63_{N74H}xb12 on whole blood cells

To demonstrate that bsHER2xCD63_{N74H} does not bind to, and accumulate in, healthy tissues that do not express the model tumor antigen HER2, bsHER2xCD63_{N74H} and monovalent and bivalent control antibodies were conjugated with FITC. Their accumulation was investigated in granulocytes and thrombocytes of healthy donors that do not express HER2. Whole blood samples from healthy donors were collected in Heparin tubes. Whole blood was diluted 1:2 in RPMI-1640 supplemented with 10% heat-inactivated cosmic calf serum. Anti-CD63 antibodies were conjugated with FITC (Thermo Scientific) according to manufacturer's instruction and added to whole blood cells at final concentration of 10 µg/mL.

Following 1 hour incubation at 4°C or 3 and 16 hours incubation at 37°C, erythrocytes were lysed by incubating 15 minutes at 4°C with erythrocyte lysis buffer (155 mM NH₄Cl, 10 mM KHCO₃ and 0.1 mM EDTA at pH 7.4). Fluorescence intensities of FITC were measured on a flow cytometer (BD). Granulocytes were gated using mouse anti-human CD66b-PerCP-Cy5.5 (BD) and thrombocytes were gated using mouse anti-human CD62-APC (BD).

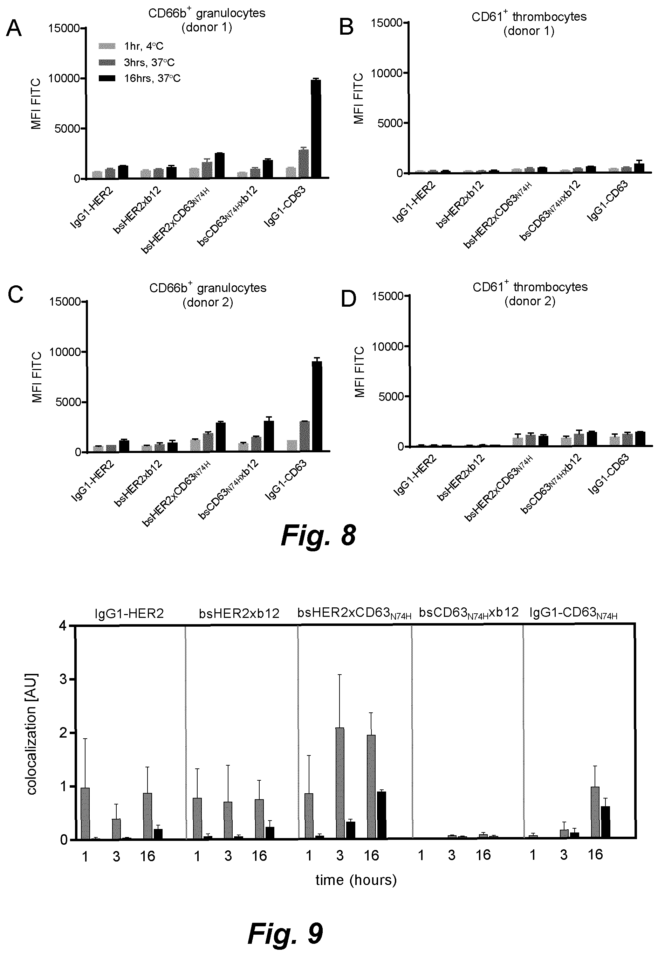

Figure 8 shows hardly any, or very low levels of, binding of CD63 antibodies to granulocytes or thrombocytes after 1 hour. However, FITC fluorescence of IgG1-CD63 on granulocytes was clearly increased after 16 hours of incubation, indicating accumulation of IgG1-CD63 into granulocytes. In contrast, FITC fluorescence of bsCD63_{N74H}xb12 and bsHER2xCD63_{N74H} was hardly increased after 16 hours (see Figure 8). Likewise IgG1-HER2 and bsHER2xb12 did not show any binding to or intracellular accumulation in granulocytes or thrombocytes, which was in line with the lack of HER2 expression on these cell types. Thus, by using a low affinity CD63-specific Fab-arm, it was possible to minimize binding and intracellular accumulation of a monovalent CD63 Ab into healthy cells.

### Example 8: Confocal microscopy, lysosomal co-localization of bsHER2xCD63_{N74H} followed over time

The internalization and lysosomal co-localization of bsHER2xCD63_{N74H} was followed over time. SK-OV-3 cells (20.000) were grown on glass coverslips (Thermo Fisher Scientific) at 37°C for 16 hours. One hour prior to antibody treatment, cells were pre-incubated with 50 µg/mL leupeptin (Sigma) to block lysosomal activity. Antibody (5 or 1 µg/mL) was added and cells were incubated for 1, 3, or 16 hours at 37°C. Cells were fixed, permeabilized, and incubated 45 min with goat anti-human IgG1-FITC (Jackson) to stain for human IgG and mouse anti-human CD107a-APC (BD) to stain for lysosomes. Hoechst (Molecular Probes, 1:10.000) was added to stain the nucleus (5 minutes at RT). Coverslips were mounted (Calbiochem) on microscope slides and imaged with a Leica SPE-II confocal microscope (Leica Microsystems) equipped with LAS-AF software. 12-bit grayscale TIFF images were analyzed for co-localization using MetaMorph^{®} software (Molecular Devices). Co-localization was depicted as arbitrary units [AU] representing the total pixel intensity of antibody overlapping with the lysosomal marker LAMP1. This value was divided by the total pixel intensity of LAMP1, to correct for differences in cell density between different images. Total IgG staining was depicted as the total pixel intensity of FITC, divided by the total pixel intensity of LAMP1.

The grey bars in Figure 9 represent total IgG staining (depicted as arbitrary units). The black bars in Figure 9 represent lysosomal co-localization (depicted as arbitrary units). IgG1-HER2 and bsHER2xb12 both showed similar staining of SK-OV-3 cells after 1, 3, and 16 hours (grey bars). A small portion of IgG1-HER2 and bsHER2xb12 showed lysosomal co-localization after 16 hours antibody exposure (black bars). The CD63 targeting antibody, IgG1-CD63_{N74H}, did not show staining (grey bars) or lysosomal co-localization (black bars) after 1 and 3 hours, however 16 hours antibody exposure resulted in Ab staining of cells which all colocalized with the lysosomal marker LAMP1. The monovalent CD63 antibody, bsCD63_{N74H}xb12, did not show mAb staining or lysosomal co-localization at any of the measured time points. On the other hand, lysosomal co-localization of bsHER2xCD63_{N74H} was gradually increased over time (black bars). Data shown are mean ± standard deviation of 3 images.

### Example 9: BsHER2xCD63_{N74H} induces downmodulation of HER2

Using a HER2 downmodulation ELISA it was investigated if the strong lysosomal targeting observed with bsHER2xCD63_{N74H,} also resulted in increased downmodulation of the targeted antigen. AU565, SK-OV-3 and Colo 205 cells were seeded (1 million cells/flask) in T25 flasks (Greiner) and incubated overnight at 37°C to obtain a confluent monolayer. Antibodies were added (10 µg/mL) and cells were cultured for another 3 days at 37°C, washed and lysed. Total protein levels were quantified using bicinchoninic acid (BCA) protein assay reagent (Pierce), according to manufacturer's instruction. Next, ELISA plates (Greiner) were coated with 1 µg/mL rabbit anti-human HER2 (Cell Signalling Technology), blocked with 2% chicken serum (Hyclone) and incubated with 50 µL cell lysate. Goat anti-human HER2-biotin (R&D, 50 ng/mL) was added to detect HER2, followed by streptavidin-poly-HRP (Sanquin, 100 ng/mL). The reaction was visualized using ABTS and stopped with oxalic acid. Fluorescence at 405 nm was measured and the amount of HER2 was expressed as a percentage relative to untreated cells.

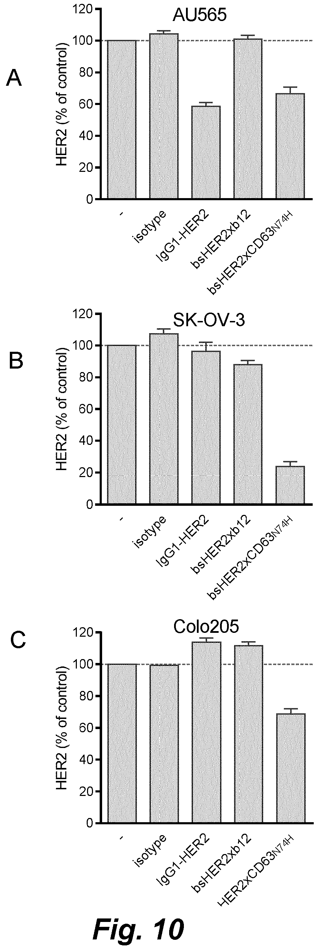

The total amount of HER2 protein in tumor cell lines with different expression levels of HER2; AU565 (500,000 HER2/cell, Figure 10A), SK-OV-3 (200,000 HER2/cell, Figure 10B) and Colo205 (50,000 HER2/cell, Figure 10C) was quantified after three days of incubation with HER2 antibody and compared with untreated cells (see Figure 10). IgG1-HER2 induced ~40% downmodulation of total HER2 in AU565 cells that express high levels of HER2. Despite the fact that the monovalent bsHER2xb12 antibody showed dose-dependent binding to HER2-positive SK-OV-3 cells (FACS binding example), no downmodulation of HER2 was observed with bsHER2xb12. This highlights that bivalent antibody binding was important for increasing the degradation of HER2. The bsHER2xCD63_{N74H} was able to restore the downmodulation of HER2 on AU565 cells. Moreover, on cell lines with lower HER2 expression, such as SK-OV-3 and Colo205, bsHER2xCD63_{N74H} also induced downmodulation of HER2, whereas IgG1-HER2 did not affect HER2 protein levels.

### Example 10: Cytotoxicity induced by Duostatin-3 conjugated ADCs

Cells were seeded in 96-well tissue culture plates (5,000 cells/well) and left to adhere for 6 hours at 37°C. Serially diluted ADCs (10-0.0005 µg/mL) were added and the cells were incubated another for 3 days at 37°C. Cell viability was assessed using CellTiter-GLO (Promega), according to the manufacturer's guidelines. The percentage of viable cells was depicted as a percentage relative to untreated cells.

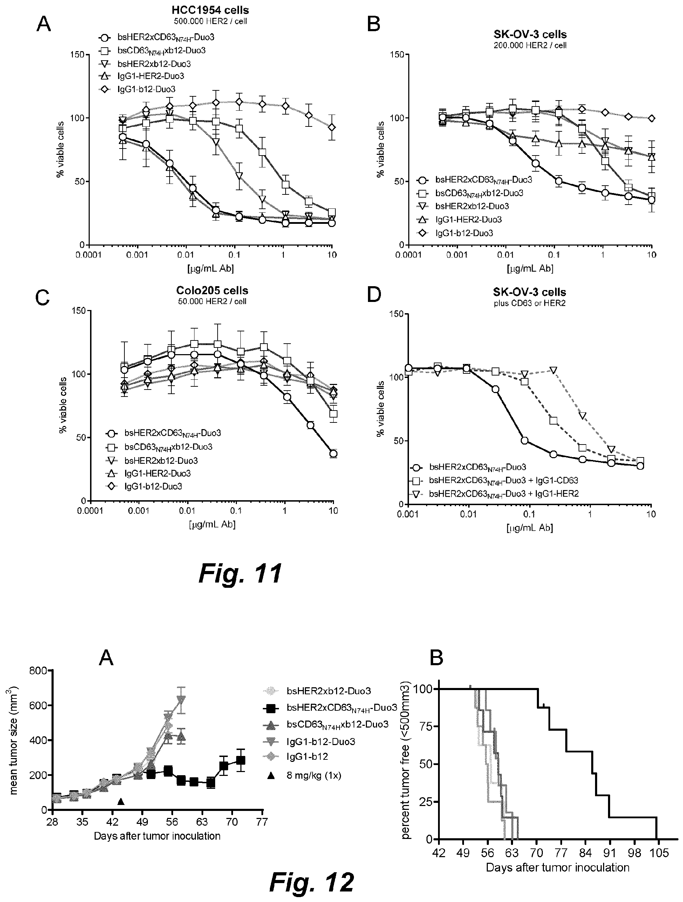

As seen in Figure 11, IgG1-HER2-Duo3 was able to kill ~80% of HCC1954 that showed high HER2 expression (500.000 HER2/cell). On SK-OV-3 cells, that express 200.000 HER2/cell, IgG1-HER2-Duo3 killed only ~30% of the cells, whereas viability of low HER2 expressing Colo205 cells (50.000 HER2/cell) was not affected.

The monovalent bsHER2xb12 killed a similar percentage of cells as compared to IgG1-HER2-Duo3, but with a ~10 fold reduced IC₅₀ value. Cytotoxicity induced by bsHER2xCD63_{N74H}-Duo3 () on HCC1954 cells was equal to IgG1-HER2-Duo3. However on cells with lower copy numbers of HER2 (SK-OV-3 and to lesser extend Colo205), bsHER2xCD63_{N74H}-Duo3 induced much more cytotoxicity as compared to ADCs only targeting HER2 (see Figure 11). Data shown are mean ± standard deviation of at least two separate experiments.

### Example 11: Anti-tumor effect of bsHER2xCD63_{N74H}-ADC on SK-OV-3 tumor xenografts

The anti-tumor effect of bsHER2xCD63_{N74H}-ADC was investigated on SK-OV-3 tumor xenografts. 6-11 week old female SCID mice (C.B-17/lcrPrkdc-scid/CRL) were purchased from Charles River. Subcutaneous tumors were induced by inoculation of 5 x 10⁶ SK-OV-3 cells in the right flank of the mice. Tumor volumes were calculated from digital caliper measurements as 0.52 x length x width² (mm³). When tumors reached 200 - 400 mm³, mice were grouped into groups of 7 mice with equal tumor size distribution and mAbs were injected intraperitoneally (8 mg/kg). During the study, blood samples were collected into heparin-containing tubes to confirm the presence of human IgG in plasma. IgG levels were quantified using a nephelometer (Siemens Healthcare). Mice that did not show human IgG in plasma were excluded from the analysis.

As shown in Figure 12, bsHER2xCD63_{N74H}-ADC induced significant inhibition of tumor growth, while the monovalent bsHER2xb12-ADC or bsCD63_{N74H}xb12-duo3, had no effect on tumor growth. This demonstrates that a low affinity CD63-specific Fab-arm can be used to induce lysosomal delivery and toxin release of a poorly internalizing ADC in tumors *in vivo.* Mantel-Cox analysis of Kalan Meyer plot indicated significant inhibition of tumor growth by bsHER2xCD63_{N74H}-ADC, P-value <0.0001.

### Example 12: Binding of bsBeta1xCD63_{N74H} to SK-OV-3 cells detected with flow cytometry

It was investigated if a low affinity binding-domain directed against E can be used to enhance internalization and lysosomal targeting of other tumor antigens as well. Integrins have been described to rely on clustering for their internalization. Therefore a monovalent integrin antibody is expected to show minimal internalization and lysosomal targeting and may therefore represent a suitable model system to test if internalization can be enhanced in a bispecific format targeting T and E. To this end, antibody huK20 that targets the integrin Beta-1 was selected. The sequence of antibody huK20 was obtained from WO1996/008564 and cloned and produced as described in Example 1 thereof.

Binding of the IgG1-Beta1 antibody, a monovalent control bsBeta1xb12 and the bispecific antibody bsBeta1xCD63_{N74H} to SK-OV-3 was investigated using flow cytometry (FACS Canto II, BD Biosciences). Serially diluted antibodies were incubated 30 minutes at 4°C with SK-OV-3 cells. Following, antibody binding was detected using a Phycoerythrin-conjugated goat-anti-human IgG antibody (Jackson) and samples were analyzed on a flow cytometer. IgG1-b12 was used as isotype control antibody.

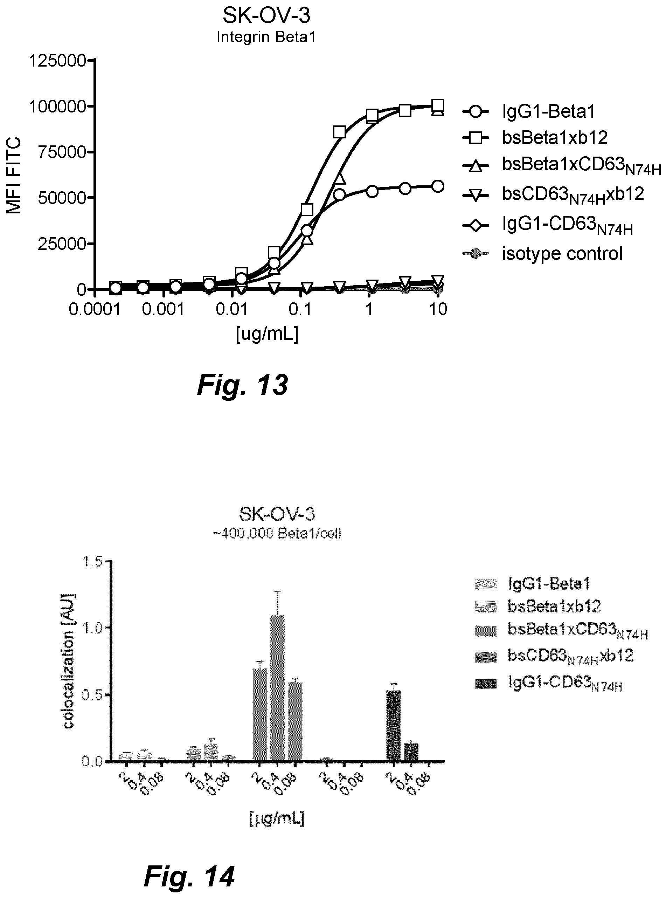

As seen in Figure 13, the binding curves of bsBeta1xCD63_{N74H} and the monovalent integrin Beta-1 antibody bsBeta1xb12, were very much alike. This indicates that tumor cell binding of bsBeta1xCD63_{N74H} occurs through monovalent binding to integrin Beta-1. IgG1-CD63_{N74H} and bsCD63_{N74H}xb12 did not show binding to SK-OV-3 cells, which is in line with the low expression of CD63 on the plasma membrane.

### Example 13: Lysosomal co-localization of bsBeta1xCD63_{N74H} measured with confocal microscopy

To investigate if dual targeting of integrin Beta-1 and CD63 results in increased lysosomal co-localization of bsBeta1xCD63_{N74H}, a confocal microscopy experiment was performed with tumor cell lines that have different copy numbers of integrin Beta-1 on the plasma membrane. 20.000 SK-OV-3, NCI-H1975 and MDA-MB-468 cells were grown on glass coverslips (Thermo Fisher Scientific) at 37°C for 4 hours. One hour prior to antibody treatment, cells were pre-incubated with 50 µg/mL leupeptin (Sigma) to block lysosomal activity. Antibody (2, 0.4, and 0.08 µg/mL) was added and cells were incubated for 16 hours at 37°C. Cells were fixed, permeabilized, and incubated 45 min with goat anti-human IgG1-FITC (Jackson) to stain for human IgG and mouse anti-human CD107a-APC (BD) to stain for lysosomes. Hoechst (Molecular Probes, 1:10.000) was added to stain the nucleus (5 minutes at RT). Coverslips were mounted (Calbiochem) on microscope slides and imaged with a Leica SPE-II confocal microscope (Leica Microsystems) equipped with LAS-AF software. 12-bit grayscale TIFF images were analyzed for co-localization using MetaMorph^{®} software (Molecular Devices). Co-localization was depicted as arbitrary units [AU] representing the total pixel intensity of antibody overlapping with the lysosomal marker LAMP1. This value was divided by the total pixel intensity of LAMP1, to correct for differences in cell density between different images.

As seen in Figure 14, bsBeta1xCD63_{N74H} demonstrated the strongest amount of lysosomal co-localization on all tested cell lines and mAb concentrations (only shown for SK-OV-3). IgG1-Beta1 and bsAb-Beta1xb12 showed modest lysosomal co-localization which was not effected by mAb concentration. IgG1-CD63_{N74H} only showed substantial lysosomal co-localization at 2 µg/mL, and 0.4 µg/mL on NCI-H1975 cells. While the monovalent control bsCD63_{N74H}xb12 only showed lysosomal co-localization at 2 µg/mL on NCI-H1975 cells which correlated with the reduced affinity of CD63_{N74H}. The increased lysosomal co-localization of bsBeta1xCD63_{N74H} was most clear on cells expressing high integrin Beta-1 copy numbers (SK-OV-3 > NCI-H1975 > MDA-MB-468). For some clones no AU were depicted because no lysosomal co-localization was measured. Data shown are mean ± standard deviation of 3 images.

### Example 14: Confocal microscopy, internalization and lysosomal co-localization of bsBeta1xCD63_{N74H} followed over time

To better understand the internalization and lysosomal co-localization kinetics of bsBeta1xCD63_{N74H}, the internalization and lysosomal co-localization of bsBeta1xCD63_{N74H} was followed over time. SK-OV-3 cells (20.000) were grown on glass coverslips (Thermo Fisher Scientific) at 37°C for 16 hours. One hour prior to antibody treatment, cells were pre-incubated with 50 µg/mL leupeptin (Sigma) to block lysosomal activity. Antibody (2 µg/mL) was added and cells were incubated for 1, 3, or 16 hours at 37°C. Cells were fixed, permeabilized, and incubated 45 min with goat anti-human IgG1-FITC (Jackson) to stain for human IgG and mouse anti-human CD107a-APC (BD) to stain for lysosomes. Hoechst (Molecular Probes, 1:10.000) was added to stain the nucleus (5 minutes at RT). Coverslips were mounted (Calbiochem) on microscope slides and imaged with a Leica SPE-II confocal microscope (Leica Microsystems) equipped with LAS-AF software. 12-bit grayscale TIFF images were analyzed for co-localization using MetaMorph^{®} software (Molecular Devices). Co-localization was depicted as arbitrary units [AU] representing the total pixel intensity of antibody overlapping with the lysosomal marker LAMP1, divided by the total pixel intensity of LAMP1. Total IgG staining was depicted as the total pixel intensity of FITC, divided by the total pixel intensity of LAMP1.

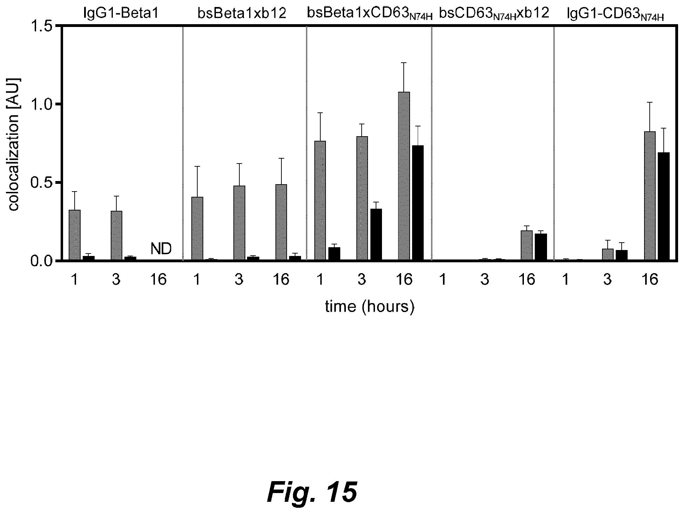

The grey bars in Figure 15 represent total IgG staining (depicted as arbitrary units). The black bars in Figure 15 represent lysosomal co-localization (depicted as arbitrary units). IgG1-Beta1 and bsBeta1xb12 both showed similar staining of SK-OV-3 cells after 1, 3, and 16 hours (grey bars), however hardly any lysosomal co-localization was measured (black bars). Thus although IgG1-Beta1 and bsBeta1xb12 were able to bind to SK-OV-3 cells, they were not transported to the lysosomes. The CD63 targeting antibodies, IgG1-CD63_{N74H} and bsCD63_{N74H}xb12, did not show staining (grey bars) or lysosomal co-localization (black bars) after 1 hour. However prolonged incubation resulted in Ab staining of cells which all colocalized with the lysosomal marker LAMP1, indicating that both antibodies were immediately transported to the lysosomes. This effect was most pronounced for IgG1-CD63_{N74H} and less pronounced for bsCD63_{N74H}xb12. Finally Beta1xCD63_{N74H} demonstrated equal staining of SK-OV-3 cells after 1, 3 and 16 hours (grey bars). While lysosomal co-localization was gradually increased over time (black bars), indicating that Beta1xCD63_{N74H} first binds to tumor cells, through integrin Beta-1, and is subsequently transported to the lysosomes. Data shown are mean ± standard deviation of at least 3 images.

## Claims

1. A multispecific antibody comprising a first antigen-binding domain and a second antigen-binding domain, wherein the first antigen-binding domain specifically binds a target molecule (T), and wherein the second antigen-binding domain specifically binds an internalizing effector protein (E), and wherein the second antigen-binding domain has a dissociation constant K_{D} value with E of between 10⁻⁹ and 10⁻⁸ M.

2. The multispecific antibody according to claim 1, wherein the second antigen-binding domain has a dissociation constant K_{D} value with E of between 2.0x10⁻⁹ and 7.3x10⁻⁹ M, the KD being determined by biolayer interferometry.

3. The multispecific antibody according to any of the preceding claims, wherein E is a cell surface-expressed molecule that is internalized into the cell, and
(a) the multispecific antibody is internalized into the cell by way of binding to E only in the presence of the target molecule (T);
(b) the multispecific antibody is internalized into the cell by way of binding to E only when the first domain is specifically bound to the target molecule (T);
(c) the multispecific antibody upon binding to E internalizes more efficiently into cells expressing T as compared to cells not expressing T;
(d) the multispecific antibody upon binding to T internalizes more efficiently into cells expressing E as compared to cells not expressing E;
(e) the multispecific antibody upon binding to E is transported to the lysosomal compartment in cells expressing T;
(f) the multispecific antibody upon binding to E is more efficiently transported to the lysosomal compartment in cells expressing T as compared to cells not expressing T;
(g) the multispecific antibody upon binding to T is more efficiently transported to the lysosomal compartment in cells expressing E as compared to cells not expressing E; and/or
(h) binding of T and E by the multispecific antibody induces internalization of the multispecific antibody to a greater extent than the binding of T alone.

4. The multispecific antibody according to any of the preceding claims, wherein E is selected from the group consisting of CD63, MHC-I, Kremen-1, Kremen-2, LRP5, LRP6, transferrin receptor, LDLr, MAL, V-ATPase and ASGR, optionally wherein E is CD63.

5. The multispecific antibody according to any of the preceding claims, wherein T is a cell surface-expressed target molecule.

6. The multispecific antibody according to any of the preceding claims, wherein T is a tumor-associated antigen.

7. The multispecific antibody according to claim 6, wherein the tumor-associated antigen includes HER2, 5T4, A33, activin receptor, adrenomedullin receptors, AFP, AGS-5, ALK, annexin, AXL, B7-H3, B7-H4, BAGE proteins, BCMA, Bombesin, C33 antigen, C4.4a, C-type lectin-like(-receptor), CA19.9, CA-125, CADM1, CAIX, CanAg, CAR, carbonic anhydrase, Caveolin-1, CCK2R, CD4, CD10, CD19, CD20, CD21, CD22, CD25, CD27, CD30, CD33, CD37, CD38, CD44, CD51, CD57, CD70, CD73, CD74, CD79a, CD79b, CD80, CEA, CEACAMs, c-kit, claudin, chemokine receptors (i.e., CXCR4, CXCR5), c-Met, Cripto-1, DEC-205, Derlin-1, Desmoglein-3, Dlk-1, DLL3, DS6, E-cadherin, E-Selectin, EAG-1, ED-B, EpCAM, EGFR, EGFRvIII, emmprin, endothelin receptor, ErbB2/Her2, ErbB3, ErbB4, ETV6-AML, Ephrin type-A receptor, Epiregulin, ETA, FAP-alpha, FcyR's, FGFR, FOLR1, Frizzled, Fyn3, Galectin, Ganglioside, GCC, GD2, GD3, GloboH, lypican-3, GLUT3, GPNMB, G-protein coupled receptors (i.e., GPR49), gp100, Hsp, HLA/B-raf, HLA-DR, HLA/k-ras, HLA MAG E-A3, HMW-MAA, hTERT, ICAM-3, IGF-R, IL-13-R, L1CAM, laminin receptor, LIV1, LMP2, LRP5, LRP6, MAGE proteins, MART-1, melanotransferrin, mesothelin, metalloproteinase, ML-IAP, Mucins, Mud, Mud 6 (CA-125), MU M1, N-cadherin, NA17, NCAM-1, Nectin4, Notch, NP-55, NRP1, NY-BR1, NY-BR62, NY-BR85, NY-ES01, PLAC1, PRLR, PRAME, prominin-1, PSMA (FOLH 1 ), RON, SLC44A4, SLITRK6, Steap-1, Steap-2, surviving, syndecan, TAG-72, TF, TGF-β, TMPRSS2, TMEFF2, TNFR, Tn, TROP2, TRP-1, TRP-2, TWEAKR, tyrosinase, uroplakin-3 and VEGFR, optionally wherein T is HER2.

8. The multispecific antibody according to any of the preceding claims, wherein the multispecific antibody is a bispecific antibody comprising a first binding arm comprising the first antigen-binding domain and a second binding arm comprising said second antigen-binding domain, optionally
(a) wherein said first antigen-binding domain comprises a first heavy chain variable sequence (VH) and a first light chain variable sequence (VL), and said second antigen-binding domain comprises a second heavy chain variable sequence (VH) and a second light chain variable sequence (VL) and wherein said variable sequences each comprise three CDR sequences, CDR1, CDR2 and CDR3;
(b) wherein (i) said first binding arm comprises a first heavy chain comprising a first heavy chain variable sequence (VH) and a first heavy chain constant sequence (CH), and a first light chain comprising a first light chain variable sequence (VL) and a first light chain constant sequence (CL), and (ii) said second binding arm comprises a second heavy chain comprising a second heavy chain variable sequence (VH) and a second heavy chain constant sequence (CH), and a second light chain comprising a second light chain variable sequence (VL) and a second light chain constant sequence (CL);
(c) wherein the bispecific antibody is a full-length antibody, preferably an IgG1 antibody.

9. The multispecific antibody according to any of the preceding claims, wherein said second domain comprises:
(a) VH CDRs 1, 2, and 3 as provided in SEQ ID Nos: 2, 3, and 4, respectively, and VL CDRs 1, 2, and 3 as provided in SEQ ID Nos: 9, 7, and 8, respectively, or
(b) VH CDRs 1, 2, and 3 as provided in SEQ ID Nos: 2, 10, and 4, respectively, and VL CDRs 1, 2, and 3 as provided in SEQ ID Nos: 6, 7, and 8, respectively, or
(c) VH CDRs 1, 2, and 3 as provided in SEQ ID Nos: 2, 11, and 4, respectively, and VL CDRs 1, 2, and 3 as provided in SEQ ID Nos: 6, 7, and 8, respectively, or
(d) VH CDRs 1, 2, and 3 as provided in SEQ ID Nos: 2, 12, and 4, respectively, and VL CDRs 1, 2, and 3 as provided in SEQ ID Nos: 6, 7, and 8, respectively,
optionally wherein said second binding domain comprises VH CDRs 1, 2, and 3 as provided in SEQ ID Nos: 2, 12, and 4, respectively, and VL CDRs 1, 2, and 3 as provided in SEQ ID Nos: 6, 7, and 8, respectively.

10. The multispecific antibody according to any of the preceding claims, wherein the antibody is a tumor-associated target (T)xCD63 bispecific antibody, optionally wherein the antibody is a HER2xCD63 bispecific antibody.

11. The multispecific antibody according to any of the preceding claims, wherein the antibody has an EC₅₀ value for binding to tumor-associated target (T)-expressing cells, such as HER2-expressing cells, of lower than 5.0 µg/ml, such as lower than 0.5 µg/ml, as determined by flow cytometry.

12. The multispecific antibody according to any of the preceding claims, wherein K_{D} is determined by biolayer interferometry at 30 °C.

13. The multispecific antibody according to any of the preceding claims, wherein the antibody is a bispecific antibody comprising:
i) a first binding arm comprising a first heavy chain comprising a first heavy chain constant sequence (CH), said first CH comprising a first CH3 region, and
ii) a second binding arm comprising a second heavy chain comprising a second heavy chain constant sequence (CH), said second CH comprising a second CH3 region,
wherein the sequences of said first and second CH3 regions are different and are such that a heterodimeric interaction between said first and second binding arm is stronger than a homodimeric interaction of each of said first and second binding arms.

14. The multispecific antibody according to claim 13, wherein in said first heavy chain CH3 region at least one of the amino acids in a position corresponding to positions T366, L368, K370, D399, F405, Y407 or K409 of human IgG1 heavy chain has been substituted, and in said second heavy chain CH3 region at least one of the amino acids in a position corresponding to positions T366, L368, K370, D399, F405, Y407 or K409 of human IgG1 heavy chain has been substituted, and wherein said first and said second heavy chains are not substituted in the same positions and wherein the amino acid positions are numbered according the EU-index, optionally wherein (i) the first CH3 region has an F405L substitution and the second CH3 region has a K409R substitution, or (ii) the first CH3 region has a K409R substitution and the second CH3 region has an F405L substitution.

15. The multispecific antibody according to any of the preceding claims, wherein the multispecific antibody is conjugated to a cytotoxic moiety, a radioisotope or a drug, optionally wherein the cytotoxic moiety is selected from the group consisting of maytansine, calicheamicin, duocarmycin, duostatin, duostatin-3, duostatin-5, rachelmycin (CC-1065), auristatin, monomethyl auristatin E, monomethyl auristatin F, doxorubicin, dolastatin, pyrrolobenzodiazepine, IGN-based toxins, alpha-amanitin, or an analog, derivative, or prodrug of any thereof.

16. A bispecific antibody fragment of the multispecific antibody according to any of the preceding claims, wherein the antibody fragment is a tandem scFv, tandem scFv-Fc, scFv-Fc knobs-into-holes, scFv-Fc-scFv, F(ab')₂, Fab-scFv, (Fab'scFv)₂, Diabody, scDiabody, scDiabody-Fc, scDiabody-C_{H}3, or azymetric scaffold.

17. The multispecific antibody according to any one of the preceding claims, for use in a method of treatment of a disease.

18. A nucleic acid encoding a multispecific antibody according to any of claims 1-16.

19. An expression vector containing the nucleic acid according to claim 18 capable of expressing said nucleic acid in prokaryotic or eukaryotic host cell lines.

20. A prokaryotic or eukaryotic host cell line comprising a vector according to claim 19.
